# EUROPEAN PATENT APPLICATION

(11) **EP 1 947 085 A1**
(43) Date of publication of application: **23.07.2008**
(21) Application number: 07384011.8
(22) Date of filing: 19.01.2007
(51) Int. Cl.: C07D 209/36, C07D 409/12, A61K 31/404, A61P 3/04

(54) **Substituted indole sulfonamide compounds, their preparation and use as medicaments**

(71) Applicant: Laboratorios del Dr. Esteve S.A., 08041 Barcelona (ES)
(72) Inventor: Beller, Matthias, 18211 Ostseebad Nienhagen (DE); Alex, Karolin, 18057 Rostock (DE); Díaz Fernández, José Luis, 08240 Manresa (ES)
(74) Representative: Carpintero Lopez, Francisco

(57) **Abstract**

The present invention relates to substituted indole sulfonamide compounds of general formula(I), 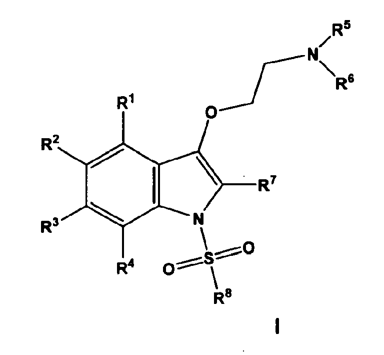 a process for their preparation, a medicament comprising these compounds and the use of substituted indole sulfonamide compounds for the preparation of medicaments for 5-HT₆ receptor regulation as well as for the treatment of disorders related thereto.

## Description

The present invention relates to substituted indole sulfonamide compounds of general formula (I), 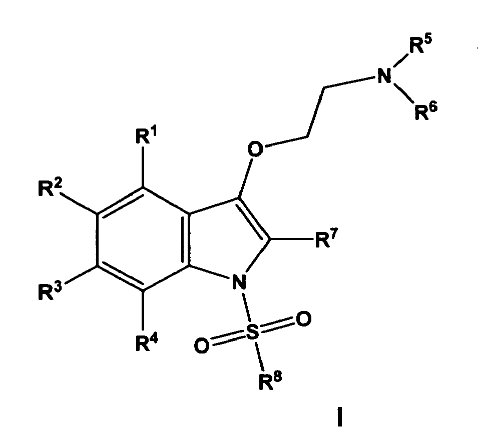 a process for their preparation, a medicament comprising these compounds and the use of substituted indole sulfonamide compounds for the preparation of medicaments for 5-HT₆ receptor regulation as well as for the treatment of disorders related thereto.

The superfamily of serotonin receptors (5-HT) includes 7 classes (5-HT₁-5-HT₇) encompassing 14 human subclasses. The 5-HT₆ receptor is the latest serotonin receptor identified by molecular cloning both in rats and in humans.

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders o

Moreover, it has been shown that the 5-HT₆ receptor also plays a role in food ingestion.

Food ingestion disorders, particularly obesity, are a serious, fast growing threat to the health of humans of all age groups, since they increase the risk of developing other serious, even life-threatening diseases such as diabetes or coronary diseases.

Thus, it was an object of the present invention to provide novel compounds that are suitable in particular as active substances in medicaments, preferably in medicaments for the regulation of 5-HT₆ receptors, for cognitive enhancement, for the prophylaxis and/or treatment of food-intake related disorders, disorders of the central nervous system, disorders of the gastrointestinal tract, such as irritable intestine syndrome, anxiety, panic, depression, cognitive memory disorders, senile dementia disorders, such as Morbus Alzheimer, Morbus Parkinson and Morbus Huntington, schizophrenia, psychosis, infantile hyperkinesia, ADHD (attention deficit, hyperactivity disorders) and other 5-HT₆ mediated disorders particularly in mammals, including humans.

It has been found that the indole-derived sulfonamide compounds of general formula (I), including compounds of general formulae (Ia), (Ib), (Ic), (Id), (Ie), (If), (Ig) and (Ih) given below show affinity for the 5-HT₆-receptor.

These compounds are therefore also suitable for the manufacture of a medicament for cognitive enhancement, for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-insulin Dependent Diabetes Mellitus), preferably type II diabetes, which is caused by obesity, disorders of the central nervous system, disorders of the gastrointestinal tract, such as irritable intestine syndrome, anxiety, panic, depression, cognitive memory disorders, senile dementia disorders, such as Morbus Alzheimer, Morbus Parkinson and Morbus Huntington, schizophrenia, psychosis, infantile hyperkinesia, ADHD (attention deficit, hyperactivity disorders) and other 5-HT₆ mediated disorders particularly in mammals, including man.

Thus, in one of its aspects the present invention relates to an indole sulfonamide compound of general formula (I), 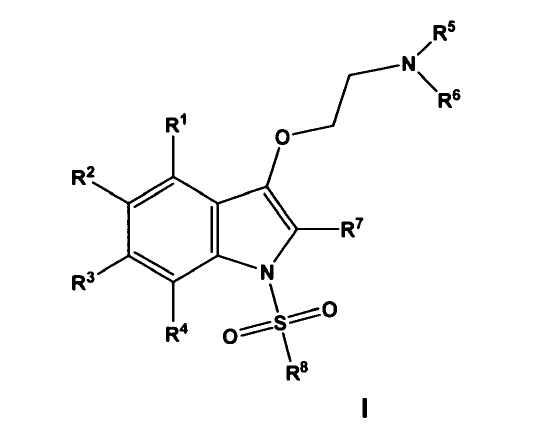 wherein
R¹, R², R³ and R⁴, independent from one another, each represents a hydrogen atom; a halogen atom; -NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁹; -S(=O)₂-R¹⁰; -OR¹¹; -SR¹²; -C(=O)-OR¹³; -NH-R¹⁶; -NR¹⁷R¹⁸; -C(=O)-NHR¹⁹; -C(=O)-NR²⁰R²¹; -S(=O)₂-NHR²²; -S(=O)₂-NR²³R²⁴; -O-C(=O)-R²⁵; -NH-C(=O)-R²⁶; -NR²⁷-C(=O)-R²⁸; -NH-C(=O)-O-R²⁹; -NR³⁰-C(=O)-O-R³¹; -S(=O)₂-O-R³²;
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted saturated, unsaturated or aromatic mono- or bicyclic ring system;
   or
an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted saturated or unsaturated or aromatic mono- or bicyclic ring system;
R⁵ and R⁶, independent from one another, each represents a hydrogen atom; or a linear or branched, saturated or unsaturated, unsubstituted or at least mono-substituted aliphatic radical;
   or
R⁵ and R⁶ together with the bridging nitrogen atom form a saturated or unsaturated, unsubstituted or at least mono-substituted, optionally at least one additional heteroatom as ring member containing heterocycloaliphatic radical, which may be condensed with an unsubstituted or at least mono-substituted saturated, unsaturated or aromatic mono- or bicyclic ring system;
   or
R⁵ and R⁶ together with the bridging nitrogen atom form an unsubstituted or at least mono-substituted, optionally at least one additional heteroatom as ring member containing heteroaryl radical, which may be condensed with an unsubstituted or at least mono-substituted saturated, unsaturated or aromatic mono- or bicyclic ring system;
R⁷ represents a hydrogen atom; or a linear or branched, saturated or unsaturated, unsubstituted or at least mono-substituted aliphatic radical;
R⁸ represents a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted saturated, unsaturated or aromatic mono- or bicyclic ring system;
an unsubstituted or at least mono-substituted phenyl radical, which is bonded via an unsubstituted or at least mono-substituted, linear or branched alkylene group; an unsubstituted or at least mono-substituted 10- or 14-membered aryl radical, which may be bonded via a linear or branched, unsubstituted or at least mono-substituted alkylene, alkenylene or alkinylene group and/or which may be condensed with an unsubstituted or at least mono-substituted, saturated, unsaturated or aromatic mono-or bicyclic ring system;
an unsubstituted or at least mono-substituted 5- or 6-membered heteroaryl radical, which may be bonded via an unsubstituted or at least mono-substituted, linear or branched alkylene, alkenylene or alkinylene group and/or which may be condensed with an unsubstituted or at least mono-substituted, saturated, unsaturated or aromatic mono- or bicyclic ring system;
or an unsubstituted or at least mono-substituted ring system selected from the group consisting of: 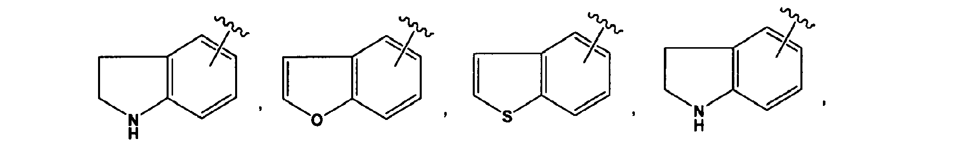 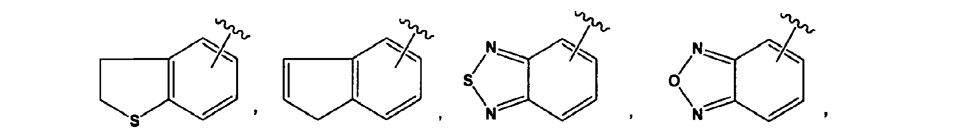 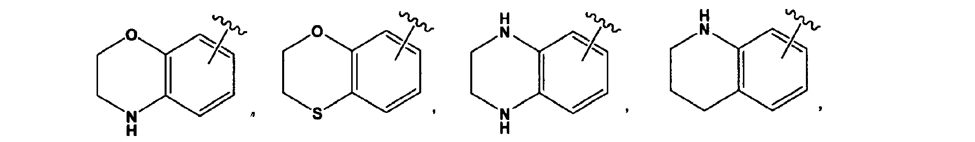 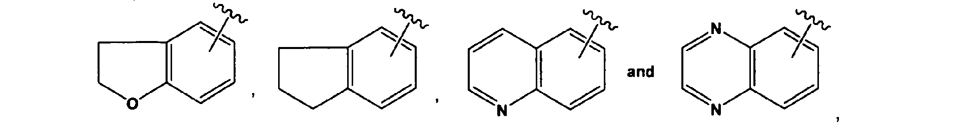
whereby these afore depicted moieties are bonded to the general formula (I) via their aromatic part;
R⁹, R¹⁰, R¹¹, R¹², R¹³, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹ and R³², independent from one another, each represents a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted saturated, unsaturated or aromatic mono- or bicyclic ring system;
   or
an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted saturated, unsaturated or aromatic mono- or bicyclic ring system;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

As a general remark, with respect to the present invention, substitution of ring systems (here exemplified by radicals R^{x}, R^{y} and R^{z} and a naphthyl ring) as indicated in Figure 1 below 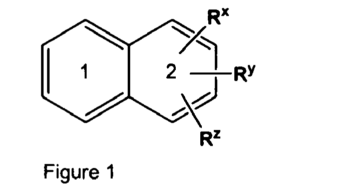 means substitution at any suitable position of the ring (in this case ring 1 and/or ring 2) by substituents R^{x}, R^{y} and R^{z}.

If any of the substituent(s) in any of the herein defined formulae represents or comprises a (hetero)cycloaliphatic radical, i.e. a cycloaliphatic radical or a heterocycloaliphatic, including a C₃₋₉ (hetero)cycloalkyl radical, i.e. a C₃₋₉ cycloalkyl or a C₃₋₉ heterocycloalkyl radical, or a C₄₋₉ (hetero)cycloalkenyl radical, i.e. a C₄₋₉ cycloalkenyl or a C₄₋₉ heterocycloalkenyl radical, said (hetero)cycloaliphatic radical, C₃₋₉ (hetero)cycloalkyl radical or C₄₋₉ (hetero)cycloalkenyl is - if not defined otherwise - unsubstituted or optionally substituted with one or more substituents, preferably unsubstituted or optionally substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -O-CFH₂, -O-CF₂H, -S-CFH₂, -S-CF₂H, -SO₃H, -NH-C(=O)-C₁₋₅-alkyl, -N(C₁₋₅-alkyl)-C(=O)-C₁₋₅-alkyl, -C(=O)-C₁₋₅-perfluoroalkyl, -S(=O)₂-NH₂, -S(=O)₂-NH-C₁₋₅-alkyl, -S(=O)₂-NH-phenyl, -NH-S(=O)₂-C₁₋₅-alkyl, cyclobutyl, cyclopropyl, cyclohexyl, cyclopentyl, cyclohexyl, pyrrolidinyl, piperidinyl, morpholinyl, phenyl, phenethyl, phenoxy and benzyl; whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituent(s) may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenethyl, phenoxy and benzyl; whereby in each occurrence the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

If any of the substituent(s) in any of the herein defined formulae represents or comprises a (hetero)cycloaliphatic radical, i.e. a cycloaliphatic or a heterocycloaliphatic radical, including a C₃₋₉ (hetero)cycloalkyl radical, i.e. a C₃₋₉ cycloalkyl radical or a C₃₋₉ heterocycloalkyl radical, or a C₄₋₉(hetero)cycloalkenyl, i.e. a C₄₋₉ cycloalkenyl or a C₄₋₉ heterocycloalkenyl radical, which contains one or more, preferably 1, 2 or 3 heteroatom(s) as ring member(s), unless defined otherwise, each of these heteroatom(s) may preferably be selected independently from the group consisting of nitrogen, oxygen and sulfur.

If any of the substituent(s) in any of the herein defined formulae represents or comprises a C₃₋₉ (hetero)cycloalkyl radical, i.e. a C₃₋₉ cycloalkyl or a C₃₋₉ heterocycloalkyl radical, or a C₄₋₉ (hetero)cycloalkenyl, i.e a C₄₋₉ cycloalkenyl or a C₄₋₉ heterocycloalkenyl radical, which contains optionally 1 or 2 additional heteroatom(s) as ring member(s), unless defined otherwise, each of these heteroatom(s) may preferably be selected independently from the group consisting of nitrogen, oxygen and sulfur.

Suitable saturated or unsaturated, optionally at least one heteroatom as ring member containing (hetero)cycloaliphatic radicals, i.e. cycloaliphatic or heterocycloaliphatic radicals, C₃₋₉ (hetero)cycloalkyl radicals, i.e. C₃₋₉ cycloalkyl or C₃₋₉ heterocycloalkyl radicals or C₄₋₉ (hetero)cycloalkenyl radicals, i.e. C₄₋₉ cycloalkenyl or C₄₋₉ heterocycloalkenyl radicals may preferably be selected from the group consisting of cyclopropyl, cyclobutyl, cyclopentyl, cyclohexyl, cycloheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, 2,5-dihydro-1H-tetrazolyl, (2,3)-dihydro-1H-pyrrolyl, (4,5)-dihydro-1 H-pyrazolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, octahydro-cyclopenta[c]pyrrolyl, (1,3)-dioxanyl, (1,4)-dioxanyl and (1,3)-dioxolanyl.

Suitable saturated or unsaturated, optionally at least one heteroatom as ring member containing (hetero)cycloaliphatic radicals, i.e. cycloaliphatic or heterocycloaliphatic radicals, C₃₋₉ (hetero)cycloalkyl radicals, i.e. C₃₋₉ cycloalkyl or C₃₋₉ heterocycloalkyl radicals or C₄₋₉ (hetero)cycloalkenyl radicals, i.e. C₄₋₉ cycloalkenyl or C₄₋₉ heterocycloalkenyl radicals, which are condensed with an unsubstituted or at least mono-substituted mono- or bicyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydrocarbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, octahydropyn-olo[1,2-a]pyrazinyl, octahydro-2H-pyrido[3,2-b][1,4]oxazinyl, octahydro-1H-cyclopenta[b]pyridinyl, dodecahydro-1H-carbazolyl, octahydrofuro[2,3-b]pyridinyl, fluorenyl, carbazolyl, (4,5,6,7)-tetrahydro-1 H-indolyl, octahydro-1H-pyrido[1,2-α]pyrazine, (3a,4,5,6,7,7a)-hexahydro-1H-pyrrolo[2,3-b]pyridinyl, (3a,4,5,6,7,7a)-hexahydrothieno[2,3-b]pyridinyl, (3a,4,5,6,7,7a)-hexahy-drofuro[2,3-b]pyridinyl, octahydrothieno[2,3-b]pyridinyl, octahydro-1H-pyrazino[1,2-a]pyrimidinyl, octahydro-1H-pyrrolo[2,3-b]pyridinyl, octahydropyrrolo[1,2-α]pyrazinyl, octahydro-1H-indolyl, octahydrocyclopenta[b]pyrrolyl and (1,2,3,4)-tetrahydroqui-noxazlinyl.

Suitable saturated or unsaturated C₃₋₉ heterocycloalkyl and C₄₋₉ heterocycloalkenyl rings may preferably be selected from the group consisting of pyrrolidinyl, piperidinyl, piperazinyl, homopiperazinyl, morpholinyl, aziridinyl, azetidinyl, imidazolidinyl, thiomorpholinyl, pyrazolidinyl, azepanyl, diazepanyl, azocanyl, (1,2,3,6)-tetrahydropyridinyl, (1,2,3,4)-tetrahydropyridinyl, (1,2,5,6)-tetrahydropyridinyl, hexahydropyrimidinyl, (5,6)-dihydro-4H-pyrimidinyl and (5,6)-dihydro-4H-pyrimidinyl.

Suitable saturated or unsaturated C₃₋₉ heterocycloalkyl and C₄₋₉ heterocycloalkenyl rings, which are condensed with an unsubstituted or at least mono-substituted mono-or bicyclic ring system may preferably be selected from the group consisting of indolinyl, isoindolinyl, (1,2,3,4)-tetrahydroquinolinyl, (1,2,3,4)-tetrahydroisoquinolinyl, octahydro-cyclopenta[c]pyrrolyl, decahydroquinolinyl, dodecahydrocarbazolyl, 9H-carbazolyl, decahydroisoquinolinyl, (2,3)-dihydro-1H-cyclopenta[b]indolyl, [1,2,3,4]-tetrahydroquinazolinyl, [3,4]-dihydro-2H-benzo[1,4]oxazinyl, octahydropyrrolo[1,2-a]pyrazinyl, octahydro-2H-pyrido[3,2-b][1,4]oxazinyl, octahydro-1 H-cyclopenta[b]pyridinyl, dodecahydro-1H-carbazolyl, octahydrofuro[2,3-b]pyridinyl, carbazolyl, (4,5,6,7)-tetrahydro-1 H-indolyl, octahydro-1H-pyrido[1,2-a]pyrazine, (3a,4,5,6,7,7a)-hexahydro-1 H-pyrrolo[2,3-b]pyridinyl, (3a,4,5,6,7,7a)-hexahy-drothieno[2,3-b]pyridinyl, (3a,4,5,6,7,7a)-hexahydrofuro[2,3-b]pyridinyl, octahy-drothieno[2,3-b]pyridinyl, octahydro-1H-pyrazino[1,2-a]pyrimidinyl, octahydro-1H-pyrrolo[2,3-b]pyridinyl, octahydro-1H-indolyl, octahydropyrrolo[1,2-α]pyrazinyl, octahy-drocyclopenta[b]pyrrolyl and (4,5,6,7)-tetrahydro-1 H-indolyl.

If any of the substituents in any of the herein defined formulae represents an alkylene group, including an C₁₋₆ alkylene group, an alkenylene group, including an C₂₋₆ alkenylene group or an alkinylene group, including an C₂₋₆ alkinylene group, said alkylene group, C₂₋₆ alkylene group, alkenylene group, C₂₋₆ alkenylene group, alkinylene group or C₂₋₆ alkinylene group is unsubstituted or substituted with one or more substituents, preferably unsubstituted or optionally substituted with 1, 2, 3, 4 or 5 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃-, -(CH₂)₄-, -(CH₂)₅ and -(CH₂)₆-, suitable alkenylene groups include -CH=CH-, -CH₂-CH=CH- and -CH=CH-CH₂- and suitable alkinylene groups include -C≡C-, -CH₂-C≡C- and -C≡C-CH₂-.

If any of the substituents in any of the herein defined formulae represents or comprises an aryl radical, including a 6-membered aryl radical such as phenyl or a 10-membered aryl radical such as naphthyl or a 14-membered aryl radical such as anthracenyl, said aryl radical is - if not defined otherwise - unsubstituted or substituted with one or more substituents, preferably unsubstituted or optionally substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -O-CFH₂, -O-CF₂H, -S-CFH₂, -S-CF₂H, -SO₃H, -NH-C(=O)-C₁₋₅-alkyl, -N(C₁₋₅-alkyl)-C(=O)-C₁₋₅-alkyl, -C(=O)-C₁₋₅-perfluoroalkyl, -S(=O)₂-NH₂, -S(=O)₂-NH-C₁₋₅-alkyl, -S(=O)₂-NH-phenyl, -NH-S(=O)₂-C₁₋₅-alkyl, cyclobutyl, cyclopropyl, cyclohexyl, cyclopentyl, cyclohexyl, pyrrolidinyl, piperidinyl, morpholinyl, phenyl, phenethyl, phenoxy and benzyl; whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

Preferred aryl radicals, which may optionally be at least mono-substituted, are phenyl and naphthyl.

If any of the substituents in any of the herein defined formulae represents or comprises a heteroaryl radical, including a monocyclic 5- or 6-membered heteroaryl radical or a bi- or tricyclic 8-, 9-, 10-, 11-, 12-, 13- or 14- membered heteroaryl radical, said heteroaryl radical is - if not defined otherwise - unsubstituted or substituted by one or more substituents, preferably unsubstituted or optionally substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -O-CFH₂, -O-CF₂H, -S-CFH₂, -S-CF₂H, -SO₃H, -NH-C(=O)-C₁₋₅-alkyl, -N(C₁₋₅-alkyl)-C(=O)-C₁₋₅-alkyl, -C(=O)-C₁₋₅-perfluoroalkyl, -S(=O)₂-NH₂, -S(=O)₂-NH-C₁₋₅-alkyl, -S(=O)₂-NH-phenyl, -NH-S(=O)₂-C₁₋₅-alkyl, cyclobutyl, cyclopropyl, cyclohexyl, cyclopentyl, cyclohexyl, pyrrolidinyl, piperidinyl, morpholinyl, phenyl, phenethyl, phenoxy and benzyl; whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2, 3 or 4 heteroatom(s).

Suitable bi- or tricyclic heteroaryl radicals, which may optionally be at least mono-substituted, may preferably be selected from the group consisting of indolyl, isoindolyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl.

Suitable mono-, bi- or tricyclic heteroaryl radicals, which are condensed with an unsubstituted or at least mono-substituted saturated or unsaturated mono- or bicyclic ring system, may preferably be selected from the group consisting of (4,5,6,7)-tetrahydro-1H-indolyl, [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

Suitable monocyclic 5- or 6-membered heteroaryl radicals, which may optionally be at least mono-substituted, may preferably be selected from the group consisting of pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl.

A mono- or bicyclic ring system according to the present invention - if not defined otherwise - means a mono- or bicyclic hydrocarbon ring system that may be saturated, unsaturated or aromatic. Each of its different rings may show a different degree of saturation, i.e. it may be saturated, unsaturated or aromatic. Optionally each of the rings of the mono- or bicyclic ring system may contain one or more, preferably 1, 2 or 3, heteroatom(s) as ring member(s), which may be independent from one another and which can preferably be selected from the group consisting of nitrogen, oxygen and sulfur. The rings of the mono- or bicyclic ring system are preferably 5-, 6- or 7-membered.

Preferably a mono-or bicyclic ring system according to the present invention is a phenyl or naphthyl ring system.

The term "condensed" according to the present invention means that a ring or ring system is attached to another ring or ring system, whereby the terms "annulated" or "annelated" are also used by those skilled in the art to designate this kind of attachment.

Such a mono- or bicyclic ring system is - if not defined otherwise - unsubstituted or substituted by one or more substituents, preferably unsubstituted or optionally substituted with 1, 2, 3, 4 or 5 substituent(s). Said substituents may preferably be selected independently from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -O-CFH₂, -O-CF₂H, -S-CFH₂, -S-CF₂H, -SO₃H, -NH-C(=O)-C₁₋₅-alkyl, -N(C₁₋₅-alkyl)-C(=O)-C₁₋₅-alkyl, -C(=O)-C₁₋₅-perfluoroalkyl, -S(=O)₂-NH₂, -S(=O)₂-NH-C₁₋₅-alkyl, -S(=O)₂-NH-phenyl, -NH-S(=O)₂-C₁₋₅-alkyl, cyclobutyl, cyclopropyl, cyclohexyl, cyclopentyl, cyclohexyl, pyrrolidinyl, piperidinyl, morpholinyl, phenyl, phenethyl, phenoxy and benzyl; whereby in each occurrence C₁-5-alkyl may be linear or branched and whereby the cyclic part(s) of said substituent(s) is unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenethyl, phenoxy and benzyl; whereby in each occurrence the cyclic part(s) of said substituent(s) is unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

If any of the substituents in any of the herein defined formulae represents a saturated or unsaturated aliphatic radical, i.e. an alkyl radical, including an C₁₋₁₀ alkyl radical; an alkenyl radical, including an C₂₋₁₀ alkenyl radical or an alkinyl radical, including an C₂₋₁₀ alkinyl radical; said aliphatic radical is - if not defined otherwise - unsubstituted or substituted with one or more substituents, preferably unsubstituted or optionally substituted with 1, 2, 3, 4 or 5 substituent(s). Said substituent(s) may preferably be selected independently from the group consisting of-O-C₁₋₅-alkyl, -S-C₁₋₅-alkyl, F, Cl, Br, I, -CN, -CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. More preferably said substituent(s) may preferably be selected independently from the group consisting of -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, -NH-CH₂-CH₂-CH₃, -NH-CH(CH₃)₂, -NH-C(CH₃)₃, -N(CH₃)₂ and -N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, neo-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

Preferred is a compound of general formula I,
wherein
R¹, R², R³ and R⁴, independent from one another, each represents a hydrogen atom; F; Cl; Br; I; -NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁹; -S(=O)₂-R¹⁰; -OR¹¹; -SR¹²; -C(=O)-OR¹³; -NH-R¹⁶; -NR¹⁷R¹⁸; -C(=O)-NHR¹⁹; -C(=O)-NR²⁰R²¹; -S(=O)₂-NHR²²; -S(=O)₂-NR²³R²⁴; -O-C(=O)-R²⁵; -NH-C(=O)-R²⁶; -NR²⁷-C(=O)-R²⁸; -NH-C(=O)-O-R²⁹; -NR³⁰-C(=O)-O-R³¹; -S(=O)₂-O-R³²;
a linear or branched, unsubstituted or at least mono-substituted C₁₋₁₀ alkyl radical, C₂₋₁₀ alkenyl radical or C₂₋₁₀ alkinyl radical;
an unsubstituted or at least mono-substituted C₃₋₉ (hetero)cycloalkyl radical, i.e. C₃₋₉ cycloalkyl or C₃₋₉ heterocycloalkyl radical, or C₄₋₉ (hetero)cycloalkenyl radical, i.e. C₄₋₉ cycloalkenyl or C₄₋₉ heterocycloalkenyl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
or
an unsubstituted or at least mono-substituted 6-, 10- or 14-membered mono-, bi- or tricyclic aryl or a 5-, 6-, 8-, 9-, 10-, 11-, 12-, 13- or 14-membered mono-, bi- or tricyclic heteroaryl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
R⁵ and R⁶, independent from one another, each represents a hydrogen atom; or a linear or branched, unsubstituted or at least mono-substituted C₁₋₁₀ alkyl radical, C₂₋₁₀ alkenyl radical or C₂₋₁₀ alkinyl radical;
or
R⁵ and R⁶ together with the bridging nitrogen atom form an optionally at least one additional heteroatom as a ring member containing C₃₋₉ heterocycloalkyl radical or C₄₋₉ heterocycloalkenyl radical, which may be condensed with an unsubstituted or at least mono-substituted saturated, unsaturated or aromatic mono- or bicyclic ring system;
or
R⁵ and R⁶ together with the bridging nitrogen atom an unsubstituted or at least mono-substituted 5-, 6-, 8-, 9-, 10-, 11-, 12-, 13- or 14-membered mono-, bi- or tricyclic heteroaryl radical, which may be condensed with an unsubstituted or at least mono-substituted saturated, unsaturated or aromatic mono- or bicyclic ring system;
R⁷ represents a hydrogen atom; or a linear or branched, unsubstituted or at least mono-substituted C₁₋₁₀ alkyl radical, C₂₋₁₀ alkenyl radical or C₂₋₁₀ alkinyl radical;
R⁸ represents an unsubstituted or at least mono-substituted C₃₋₉ (hetero)cycloalkyl radical, i.e. C₃₋₉ cycloalkyl or C₃₋₉ heterocycloalkyl radical, or C₄₋₉ (hetero)cycloalkenyl radical, i.e. C₄₋₉ cycloalkenyl or C₄₋₉ heterocycloalkenyl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
an unsubstituted or at least mono-substituted phenyl radical, which is bonded via a linear or branched, unsubstituted or at least mono-substituted C₁₋₆ alkylene, C₂₋₆ alkenylene or C₂₋₆ alkinylene group;
an unsubstituted or at least mono-substituted, 10- or 14-membered bi- or tricyclic aryl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted, saturated, unsaturated or aromatic mono- or bicyclic ring system;
an unsubstituted or at least mono-substituted 5- or 6-membered monocyclic heteroaryl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted, saturated, unsaturated or aromatic mono- or bicyclic ring system;
or
an unsubstituted or at least mono-substituted ring system selected from the group consisting of: 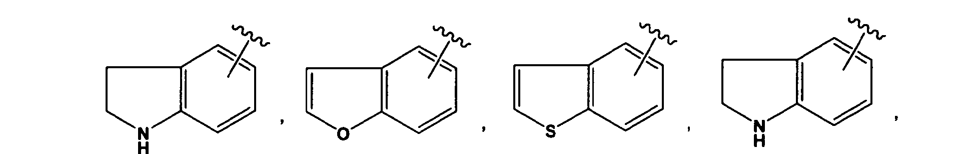 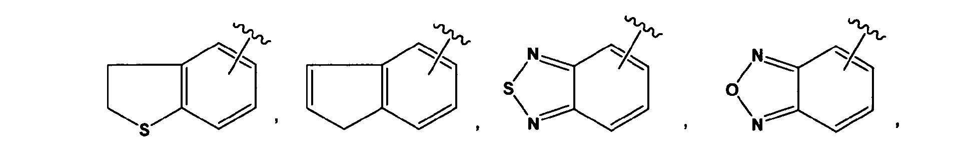 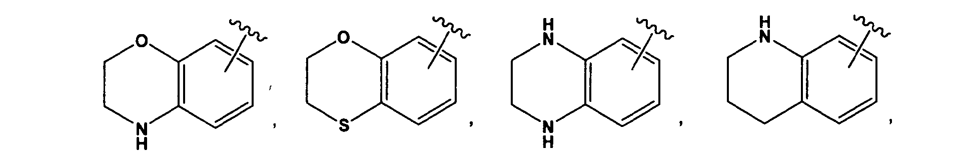 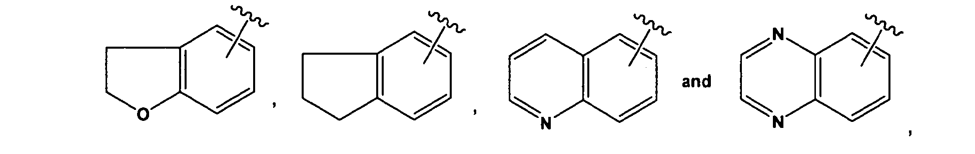 whereby these afore depicted ring systems are bonded to the general formula (I) via their aromatic phenyl part;
R⁹, R¹⁰, R¹¹, R¹², R¹³, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹ and R³², independent from one another, each represents a linear or branched, unsubstituted or at least mono-substituted C₁₋₁₀ alkyl radical, C₂₋₁₀ alkenyl radical or C₂₋₁₀ alkinyl radical;
an unsubstituted or at least mono-substituted C₃₋₉ (hetero)cycloalkyl radical, i.e. C₃₋₉ cycloalkyl or C₃₋₉ heterocycloalkyl radical, or C₄₋₉ (hetero)cycloalkenyl radical, i.e. C₄₋₉ cycloalkenyl or C₄₋₉ heterocycloalkenyl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
or
an unsubstituted or at least mono-substituted 6-, 10- or 14-membered mono-, bi- or tricyclic aryl or a 5-, 6-, 8-, 9-, 10-, 11-, 12-, 13- or 14-membered mono-, bi- or tricyclic heteroaryl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
whereby
the aforementioned C₁₋₁₀ alkyl radicals, C₂₋₁₀ alkenyl radicals or C₂₋₁₀ alkinyl radicals may in each case be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; whereby in each occurrence C₁₋₁₀-alkyl, C₂₋₁₀ alkenyl or C₂₋₁₀ alkinyl may be linear or branched;
the aforementioned C₃₋₉ (hetero)cycloalkyl radicals, i.e. C₃₋₉ cycloalkyl or C₃₋₉ heterocycloalkyl radicals, or C₄₋₉ (hetero)cycloalkenyl radicals, i.e. C₄₋₉ cycloalkenyl or C₄₋₉ heterocycloalkenyl radicals may in each case be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenethyl, phenoxy and benzyl, whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, I -CN, -CF₃, -OCF₃, -SCF₃, -OH, -SH, -NH₂ and -NO₂;
the aforementioned C₃₋₉ heterocycloalkyl or C₄₋₉ heterocycloalkenyl radicals may optionally contain 1 or 2 additional heteroatom(s) independently selected from the group consisting of nitrogen, oxygen and sulfur as ring member(s);
the aforementioned C₃₋₉ cycloalkyl and C₄₋₉ cycloalkenyl radicals in each case may optionally contain 1, 2 or 3 heteroatom(s) independently selected from the group consisting of nitrogen, oxygen and sulfur as ring member(s);
the aforementioned C₁₋₆ alkylene, C₂₋₆ alkenylene or C₂₋₆ alkinylene groups may in each case be unsubstituted or optionally substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
the rings of the aforementioned ring systems are in each case independently of one another 5-, 6-, 7-, 8-, 9,- or 10-membered and may in each case independently of one another optionally contain 1, 2 or 3 heteroatom(s) independently selected from the group consisting of nitrogen, oxygen and sulphur;
and the rings of the aforementioned ring systems may in each case be unsubstituted or optionally substituted with 1, 2, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenethyl, phenoxy and benzyl, whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, I, -CN, -CF₃, -OCF₃, -SCF₃, -OH, -SH, -NH₂ and -NO₂;
the 6-, 10- or 14-membered mono-, bi- or tricyclic aryl or 5-, 6-, 8-, 9-, 10-, 11-, 12-, 13- or 14-membered mono-, bi- or tricyclic heteroaryl radicals are unsubstituted or optionally substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenethyl, phenoxy and benzyl, whereby in each occurrence C₁₋₅-alkyl may be linear or branched and whereby the cyclic part(s) of said substituent(s) is unsubstituted or optionally substituted by 1, 2 or 3 substituent(s) independently selected from the group consisting of methyl, ethyl, n-propyl, isopropyl, methoxy, ethoxy, F, Cl, Br, I, -CN, -CF₃, -OCF₃, -SCF₃, -OH, -SH, -NH₂ and -NO₂;
and the aforementioned 5-, 6-, 8-, 9-, 10-, 11-, 12-, 13- or 14-membered mono-, bi- or tricyclic heteroaryl radicals in each case contain 1, 2, 3 or 4 heteroatom(s) independently selected from the group consisting of nitrogen, oxygen and sulfur as ring member(s);
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula I,
wherein
R¹, R², R³ and R⁴, independent from one another, each represents a hydrogen atom; F; Cl; Br; I; -NO₂; -SH; -OH; -CN; -CF₃; -OCF₃; -SCF₃; -NH₂; -O-CH₃; -O-C₂H₅; -S-CH₃; -S-C₂H₅; -NH-CH₃; -N(-CH₃)₂; -C(=O)-OH; -C(=O)-H; -S(=O)₂-OH; -C(=O)-NH₂; -S(=O)₂-NH₂;
a radical selected from the group consisting of methyl, ethyl, n-propyl, isopropyl, n-butyl, sec-butyl, isobutyl, tert-butyl, n-pentyl, neo-pentyl, n-hexyl, vinyl, allyl and ethinyl, which is unsubstituted or optionally substituted with 1, 2, 3, 4, or 5 substituents independently selected from the group consisting of F, Cl, Br, I, -OH, -SH, NH₂, -O-CH₃, -O-C₂H₅, -S-CH₃, -NH-CH₃ and -N(-CH₃)₂;
a (hetero)cycloaliphatic radical selected from the group consisting of cyclopropyl, cyclobutyl, cyclopentyl, cyclohexyl, cycloheptyl, imidazolidinyl, aziridinyl, azetidinyl, pyrrolidinyl, piperidinyl, morpholinyl, thiomorpholinyl, piperazinyl and pyrazolidinyl, which is unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, phenyl, benzyl, phenethyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂ and -NH-CH₃;
or
an aryl or heteroaryl radical selected from the group consisting of phenyl, naphthyl, thienyl (thiophenyl), pyrrolyl, imidazolyl, pyrazolyl, pyridinyl, pyridazinyl, pyrimidinyl, pyrazinyl, quinolinyl, isoquinolinyl and imidazo[2,1-b]thiazolyl, which is unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, phenyl, benzyl, phenoxy, phenethyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂ and -NH-CH₃;
R⁵ and R⁶, independent from one another, each represents a hydrogen atom or a radical selected from the group consisting of methyl, ethyl, n-propyl, isopropyl, n-butyl, sec-butyl, isobutyl, tert-butyl, n-pentyl, neo-pentyl, n-hexyl, vinyl, allyl and ethinyl, which is unsubstituted or optionally substituted with 1, 2, 3, 4, or 5 substituent(s) independently selected from the group consisting of F, Cl, Br, I, -OH and -SH;
or
R⁵ and R⁶ together with the bridging nitrogen atom form a ring system selected from the group consisting of: 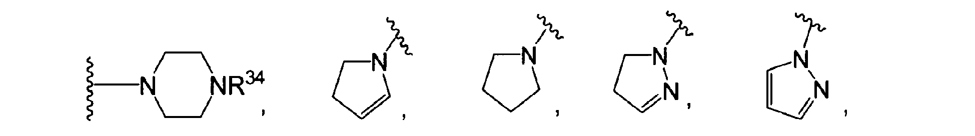 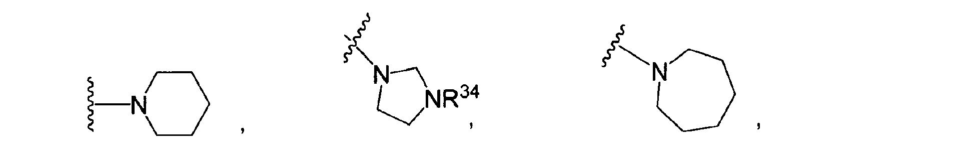 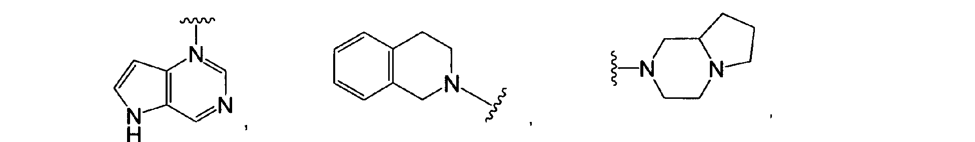 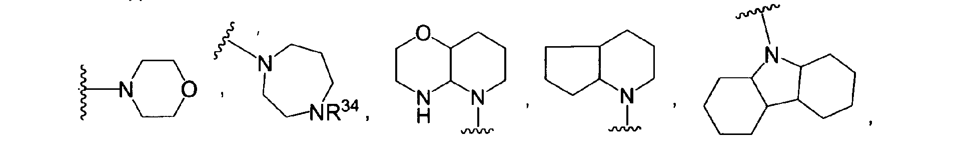 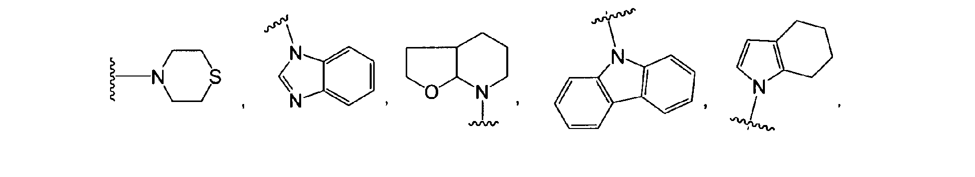 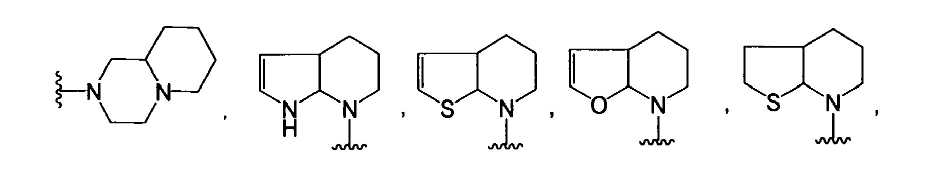 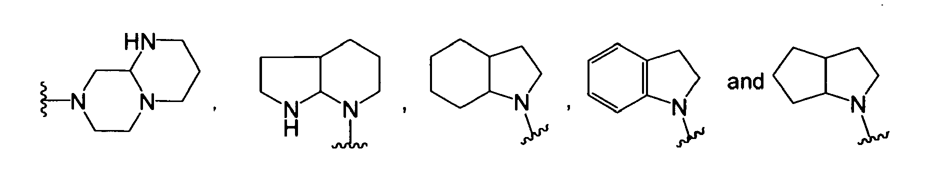 which is unsubstituted or optionally substituted in any suitable position with 1, 2, 3, 4 or 5 substituent(s) independently selected from the group consisting of oxo (=O), methyl, ethyl, n-propyl, isopropyl, n-butyl, tert-butyl, sec-butyl, phenyl, phenethyl, benzyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃, whereby the cyclic part(s) of said substituent(s) is unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂;
R³⁴ represents a hydrogen atom; methyl; ethyl; n-propyl; isopropyl; phenyl; benzyl; phenethyl; -CF₃; -CHF₂; -CH₂F; -C(=O)-CH₃; -C(=O)-C₂H₅; -C(=O)-CH₂-CH₂-CH₃; -C(=O)-NH-CH₃ or a -C(=O)-N(CH₃)₂ moiety;
R⁷ represents a hydrogen atom or a methyl, ethyl, n-propyl, isopropyl, n-butyl, sec-butyl, isobutyl or tert-butyl moiety, which is unsubstituted or optionally substituted with 1, 2, 3, 4 or 5 substituent(s) independently selected from the group consisting of F, Cl, Br, I, -CN, -NH₂, -OH and -SH;
R⁸ represents a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, azepanyl (1,2,3,6)-tetrahydropyridiny), (1,2,3,4)-tetrahydropyridinyl, (1,2,5,6)-tetrahydropyridinyl, hexahydropyrimidinyl, (5,6)-dihydro-4H-pyrimidinyl, indolinyl, isoindolinyl, (1,2,3,4)-tetrahydroquinolinyl, (1,2,3,4)-tetrahydroisoquinolinyl and octahydro-cyclopenta[c]pyrrolyl, which is unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, phenyl, benzyl, phenethyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃, whereby the cyclic part(s) of said substituent(s) is unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂;
or a ring system selected from the group consisting of: 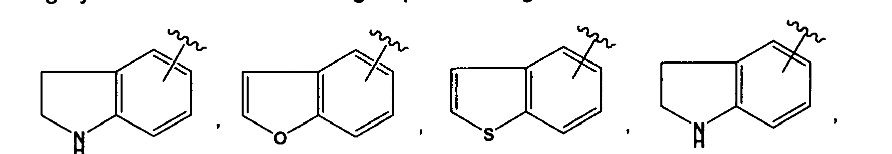 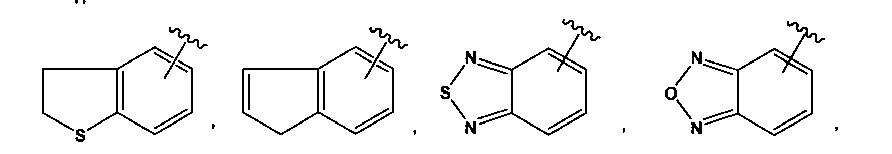 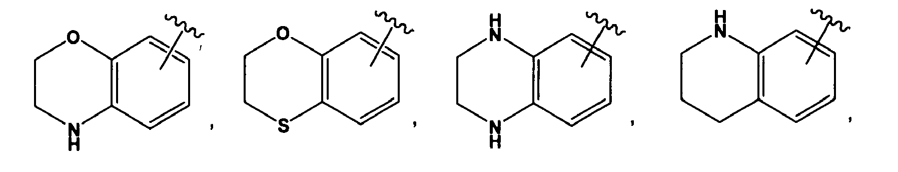 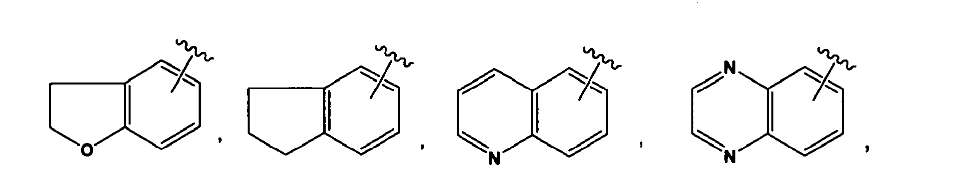 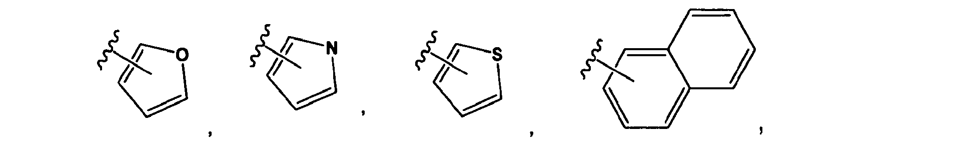 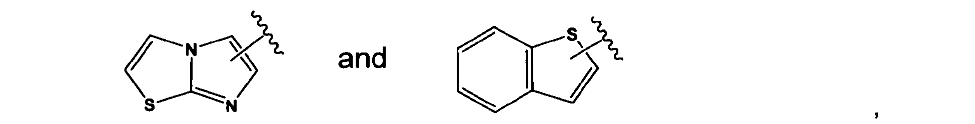 which is unsubstituted or optionally substituted in any suitable position with 1, 2, 3, 4 or 5 substituent(s) independently selected from the group consisting of oxo (=O), methyl, ethyl, n-propyl, isopropyl, n-butyl, tert-butyl, sec-butyl, phenyl, phenethyl, benzyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=0)-N(CH₃)₂, -C(=O)-N(C₂H₅)₂ and -S(=O)₂-CH₃, whereby the cyclic part(s) of said substituent(s) is unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula I,
wherein
R¹, R², R³ and R⁴, independent from one another, each represents H; F; Cl; Br; I; -NO₂; -SH; -OH; -CN; -OCF₃; -SCF₃; -NH₂; -O-CH₃; -O-CH₃-CH₃; -S-CH₃; -S-CH₂-CH₃; -NH-CH₃ or -N(CH₃)₂;
R⁵ and R⁶, independent from one another, each represents a hydrogen atom or a methyl, ethyl or n-propyl moiety which is unsubstituted or optionally substituted with 1, 2, 3, 4 or 5 substituent(s) independently selected from the group consisting of F, Cl, Br, I, -OH and -SH;
or
R⁵ and R⁶ together with the bridging nitrogen atom form a heterocyclic ring system selected from the group consisting of 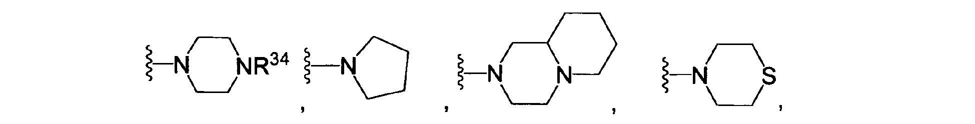 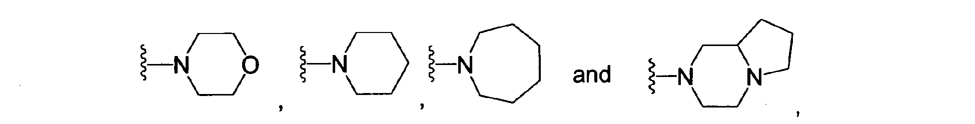 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, methyl, ethyl, phenyl, benzyl, phenethyl, -OCF₃, -SCF₃, -OH, -SH, -NH₂ and -O-CH₃;
R³⁴ represents a hydrogen atom; methyl; ethyl; n-propyl; isopropyl; phenyl; benzyl; phenethyl; -CF₃; -CHF₂; -CH₂F; -C(=O)-CH₃; -C(=O)-C₂H₅; -C(=O)-CH₂-CH₂-CH₃; -C(=O)-NH-CH₃ or a -C(=O)-N(CH₃)₂ moiety;
R⁷ represents a hydrogen atom; a methyl or an ethyl radical, which is unsubstituted or optionally substituted with 1, 2, 3, 4 or 5 substituent(s) independently selected from the group consisting of F, Cl, Br and I;
R⁸ represents a ring system selected from the group consisting of 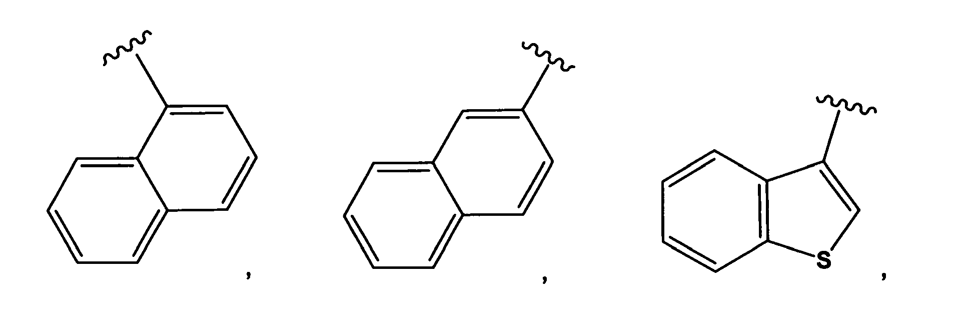 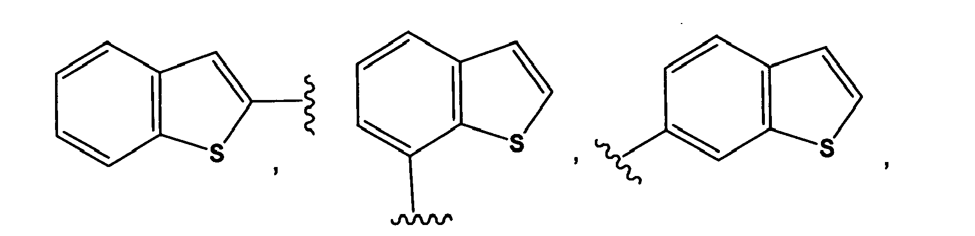 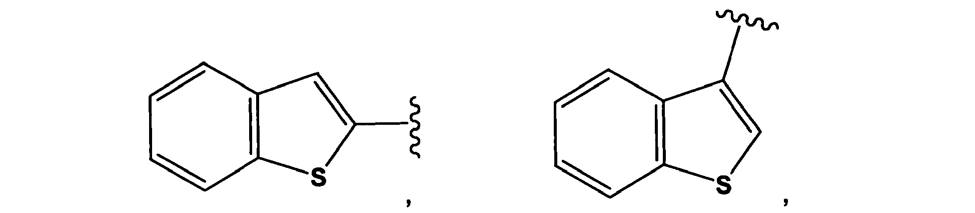 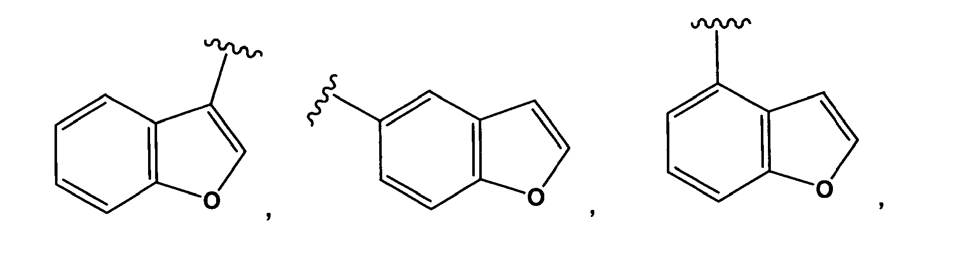 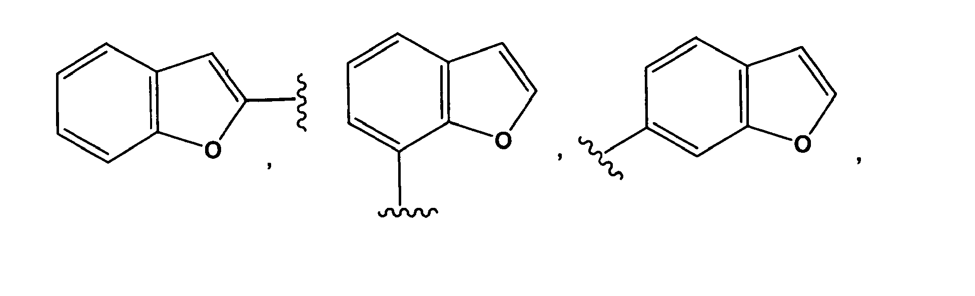 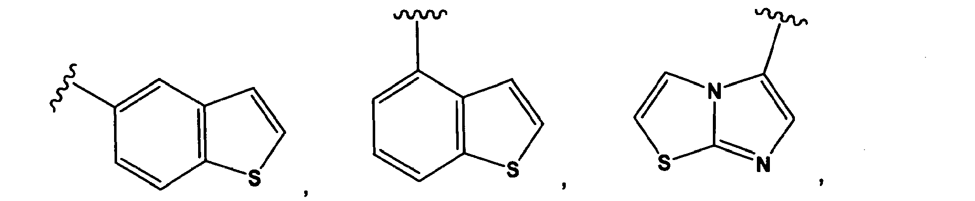 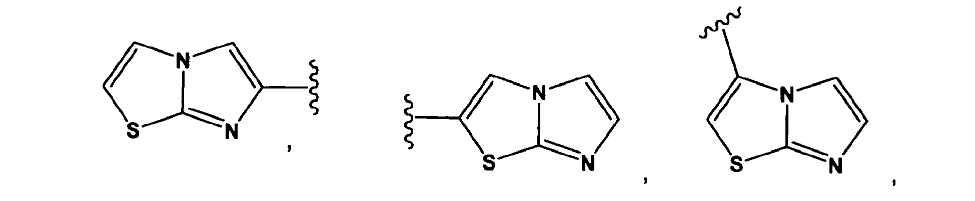 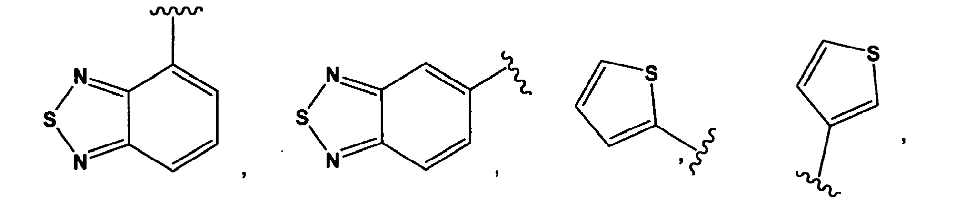 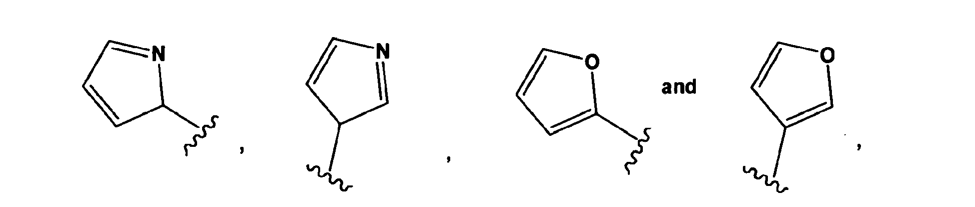 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, -methyl, -ethyl, -OCF₃, -SCF₃, -OH, -SH, -NH₂, -O-CH₃, -O-C₂H₅, -S-CH₃, -S-C₂H₅, -NH-CH₂, N(C₂H₅)₂, phenyl, phenethyl and benzyl, whereby the cyclic part(s) of said substituent(s) is unsubstituted or optionally substituted with 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula (I),
wherein
R¹, R², R³ and R⁴, independent from one another, each represents H; F; Cl; Br; I; - NO₂; -SH; -OH or -CN;
R⁵ and R⁶, independent from one another, each represents a hydrogen atom; a methyl, ethyl or n-propyl moiety which is unsubstituted or optionally substituted with 1, 2, 3, 4 or 5 substituent(s) independently selected from the group consisting of F, Cl, Br, I, -OH and -SH;
or
R⁵ and R⁶ together with the bridging nitrogen atom form a heterocyclic ring system selected from the group consisting of: 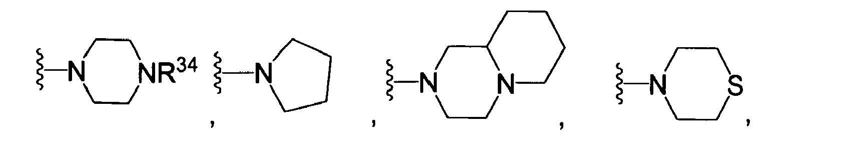 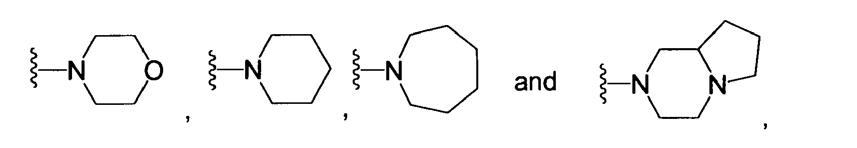 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, methyl, ethyl, phenyl, benzyl, phenethyl, -OCF₃, -SCF₃, -OH, -SH, -NH₂ and -O-CH₃,
R³⁴ represents a hydrogen atom, a methyl, ethyl or n-propyl radical;
R⁷ represents a hydrogen atom, a methyl or an ethyl radical, which is unsubstituted or optionally substituted with 1, 2 or 3 substituent(s) independently selected from the group consisting of F, Cl, Br and I;
R⁸ represents a ring system selected from the group consisting of: 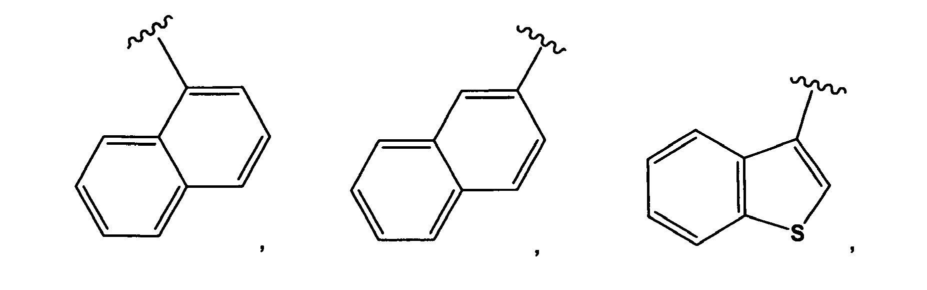 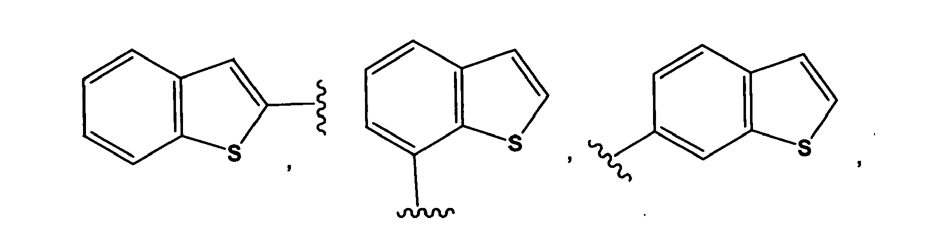 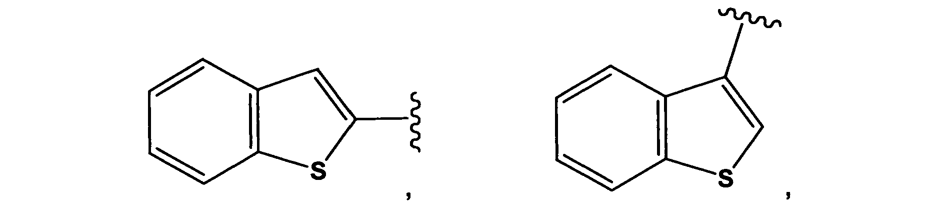 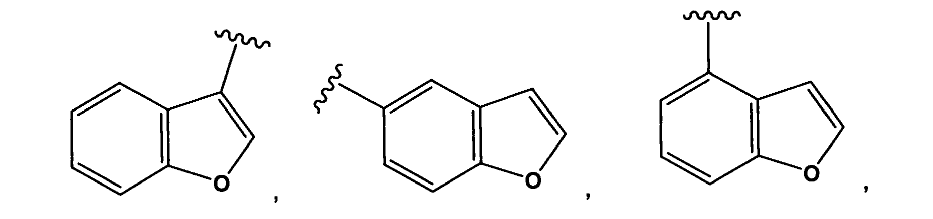 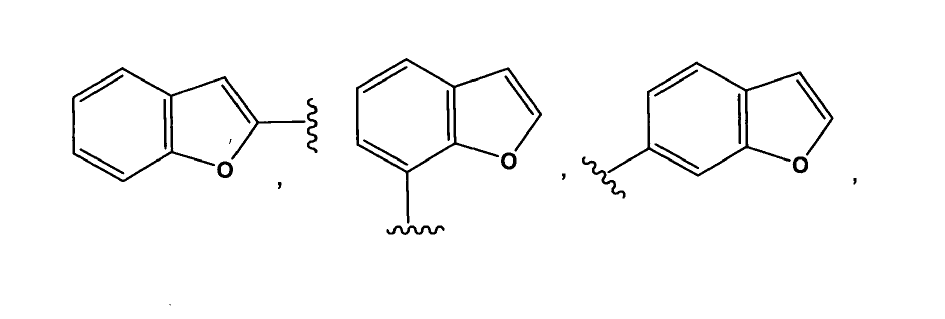 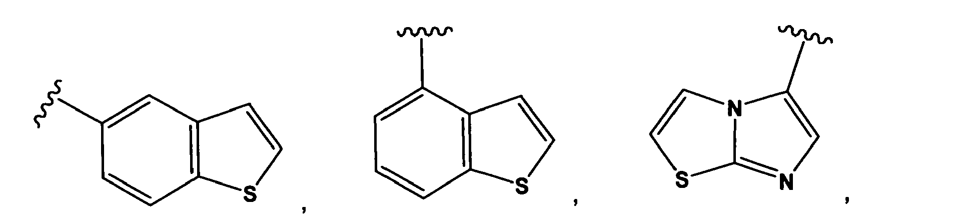 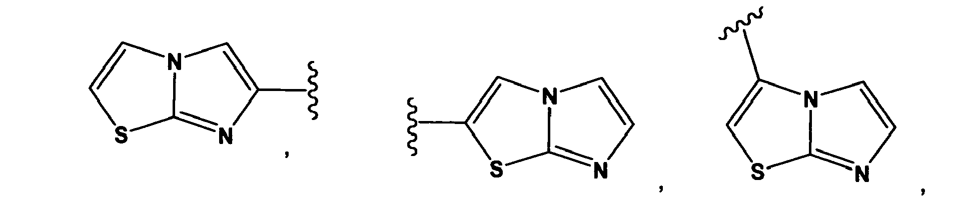 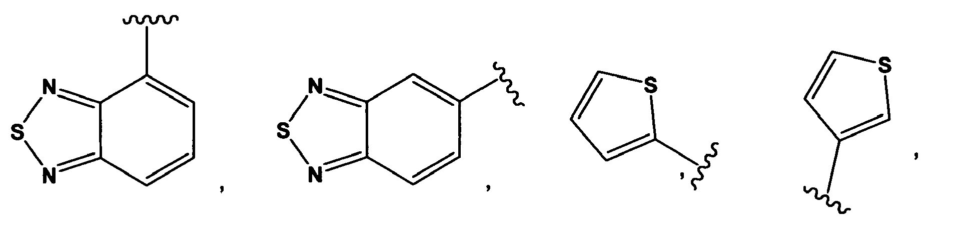 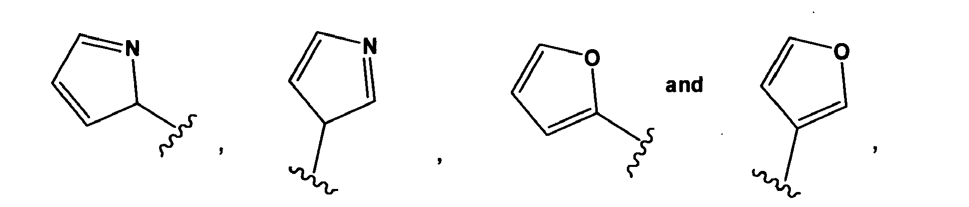 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, methyl, ethyl, phenyl, phenethyl, benzyl, -OCF₃, -SCF₃, -OH, -SH, -NH₂, -O-CH₃, -O-C₂H₅, -S-CH₃, -S-C₂H₅, -NH-CH₂ and -N(C₂H₅)₂;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula (Ia), 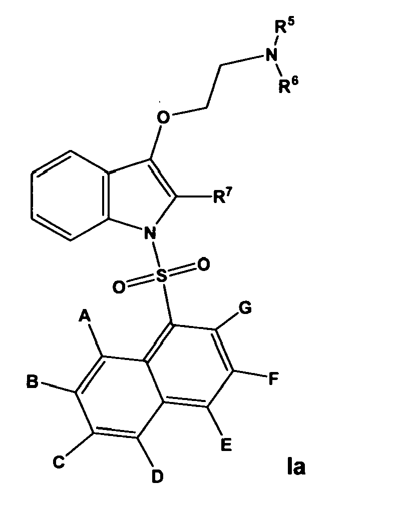 wherein
R⁵ and R⁶, independent from one another, each represents a hydrogen atom; a methyl, ethyl or n-propyl radical, which is unsubstituted or optionally substituted with 1, 2, 3, 4 or 5 substituent(s) independently selected from the group consisting of F, Cl, Br, I, -OH and -SH;
or
R⁵ and R⁶ together with the bridging nitrogen atom form a heterocyclic ring system selected from the group consisting of 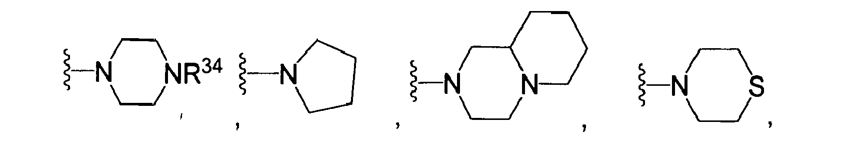 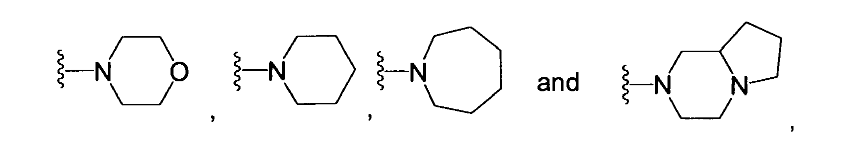 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, methyl, ethyl, phenyl, benzyl, phenethyl, -OCF₃, -SCF₃, -OH, -SH, -NH₂ and -O-CH₃,
R³⁴ represents a hydrogen atom, a methyl, ethyl or n-propyl radical;
R⁷ represents a hydrogen atom; a methyl or an ethyl radical, which is unsubstituted or optionally substituted with 1, 2, or 3 substituent(s) independently selected from the group consisting of F, Cl, Br and I;
A, B, C, D, E, F and G, independent from one another, each represents H; F; Cl; Br; I; -CN; -CF₃; -CHF₂; -CH₂F; methyl; ethyl; -OCF₃; -SCF₃; -OH; -SH; -NH₂; -O-CH₃; phenyl; benzyl or phenethyl;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula (Ib), 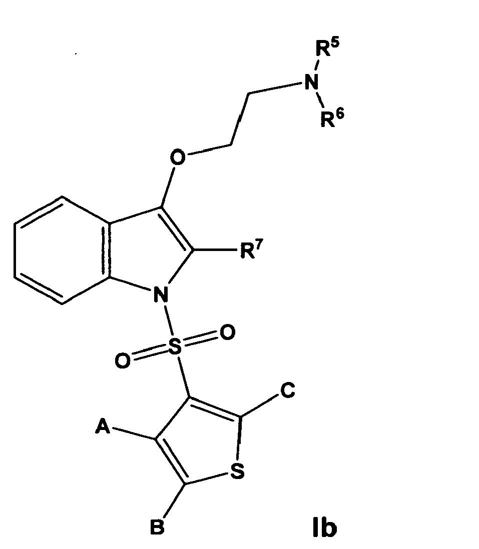 wherein
R⁵, R⁶, R⁷ and R³⁴ have the above defined meaning;
A, B, and C have the above defined meaning;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula (Ic), 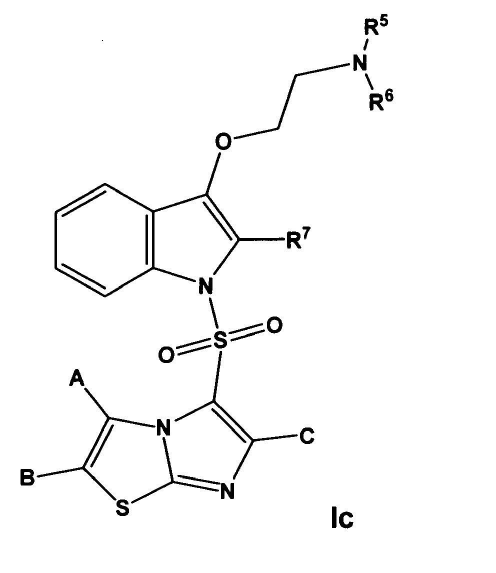 wherein
R⁵, R⁶, R⁷ and R³⁴ have the above defined meaning;
A, B, and C have the above defined meaning;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula (Id), 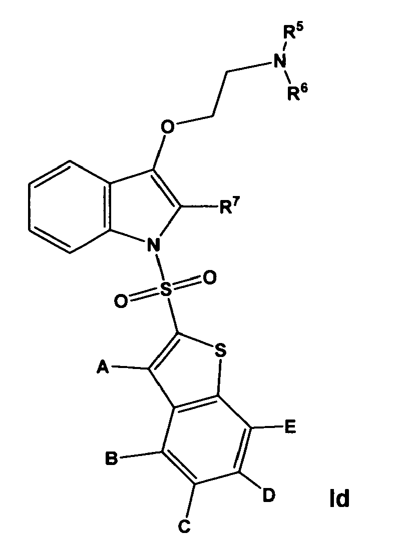 wherein
R⁵, R⁶, R⁷ and R³⁴ have the above defined meaning;
A, B, C, D and E have the above defined meaning;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula (Ie), 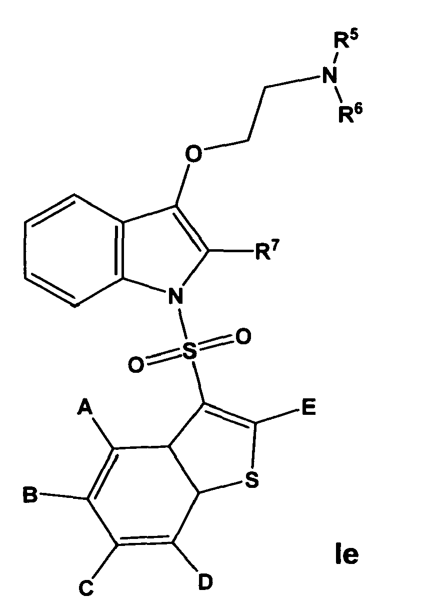 wherein
R⁵, R⁶, R⁷ and R³⁴ have the above defined meaning;
A, B, C, D, E have the above defined meaning;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula (If), 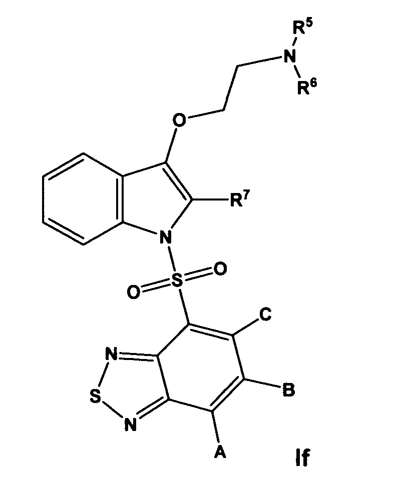 wherein
R⁵, R⁶, R⁷ and R³⁴ have the above defined meaning;
A, B, and C have the above defined meaning;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred is a compound of general formula (Ig), 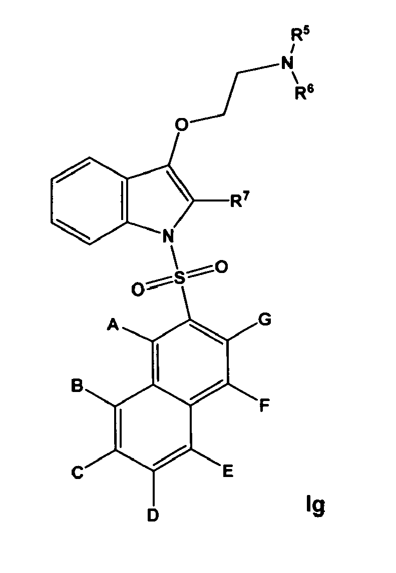 wherein
R⁵, R⁶, R⁷ and R³⁴ have the above defined meaning;
A, B, C, D, E, F and G have the above defined meaning;
optionally in the form of one of its stereoisomers, preferably enantiomers or diastereomers, its racemate or in the form of a mixture of at least two of its stereoisomers, preferably enantiomers or diastereomers, in any mixture ratio, or a salt, preferably a corresponding, physiologically acceptable salt, or a corresponding solvate.

Also preferred are compounds of general formula (Ih), 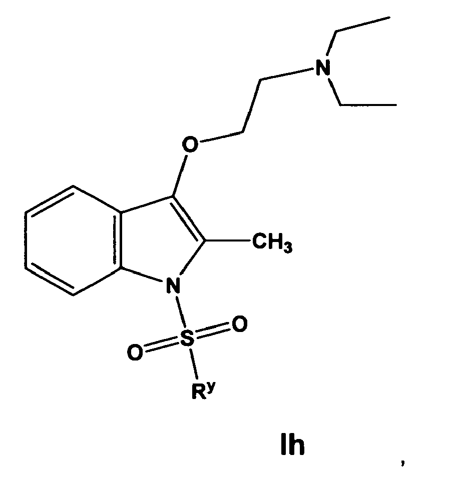 wherein
R^{y} represents a rings system selected from the group consisting of 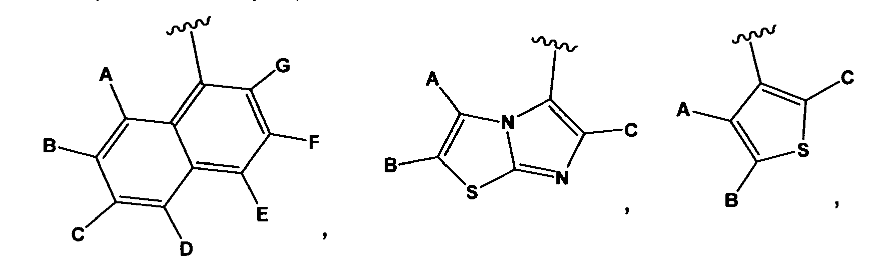 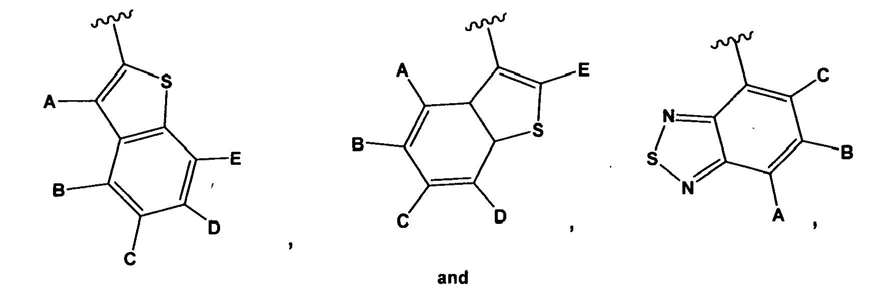 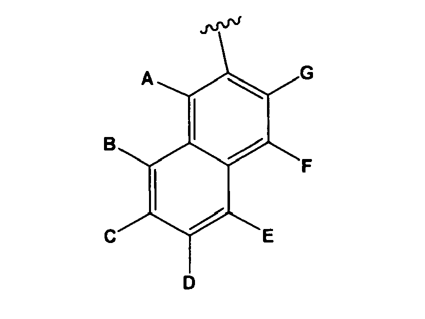 A, B, C, D, E, F and G, independent from one another, each represents H; F; Cl; Br; I; -CN; -CF₃; -CHF₂; -CH₂F; methyl; ethyl; -OCF₃; -SCF₃; -OH; -SH; -NH₂; -O-CH₃; phenyl; benzyl or phenethyl;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

Also preferred is a compound of general formula (I) selected from the group consisting of:
[1] N,N-diethyl-2-(2-methyl-1-(naphthalen-1-ylsulfonyl)-1H-indol-3-yloxy)ethanamine
[2] N,N-diethyl-2-(2-methyl-1-(naphthalen-2-ylsulfonyl)-1H-indol-3-yloxy)ethanamine
[3] 2-(1-(4-chlorothiophen-3-ylsulfonyl)-2-methyl-1H-indol-3-yloxy)-N,N-diethylethanamine
[4] 2-(1-(5-chloro-3-methylbenzo[b]thiophen-2-ylsulfonyl)-2-methyl-1H-indol-3-yloxy)-N,N-diethylethanamine
[5] 2-(1-(3a,7a-dihydrobenzo[b]thiophen-3-ylsulfonyl)-2-methyl-1H-indol-3-yloxy)-N, N-diethylethanamine
[6] 2-(1-(benzo[c][1,2,5]thiadiazol-4-ylsulfonyl)-2-methyl-1H-indol-3-yloxy)-N,N-di-ethylethanamine
[7] 2-(1-(6-chloroimidazo[2,1-b]thiazol-5-ylsulfonyl)-2-methyl-1H-indol-3-yloxy)-N,N-diethylethanamine,
and their corresponding salts and/or solvates.

Another aspect of the present invention relates to a process for the preparation of at least one substituted indole sulfonamide compound of general formula (I),
wherein at least one compound of general formula (VII) 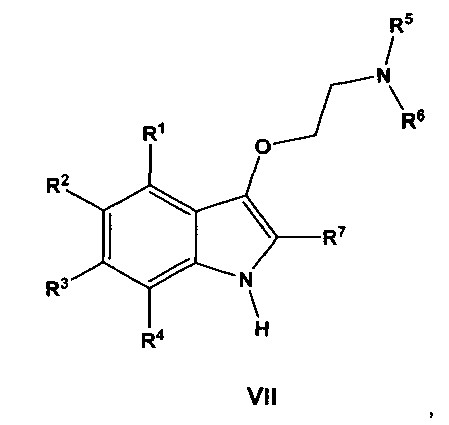 wherein R¹, R², R³, R⁴, R⁵, R⁶ and R⁷ have the meaning as defined above, which is reacted with at least one compound of general formula (VIII),

**R⁸SO₂X** **VIII,**

wherein R⁸ has the respective meaning as defined above and X represents a leaving group preferably selected from the group consisting of Cl and Br, optionally in the presence of at least one base, preferably in the presence of at least one base selected from the group consisting of alkali metal hydrides, more preferably in the presence of NaH, for prolonged time, preferably the duration of 1 to 12 h, more preferably 2 to 6 h, at an elevated temperature, preferably from 30 to 120°C, more preferably 40 to 100°C to yield at least one compound of general formula (I), 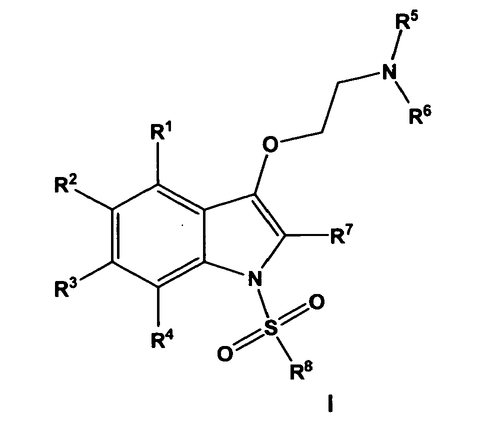 wherein R¹, R², R³, R⁴, R⁵, R⁶, R⁷ and R⁸ have the meaning as defined above.

Preferably, the aforementioned reaction is carried out in a solvent, preferably THF, DMF, dioxane, DMAc, NMP, diglyme, or ethers.

At least one substituted indole sulfonamide compound of general formula (I) given above is obtained by reacting at least one compound of general formula II, 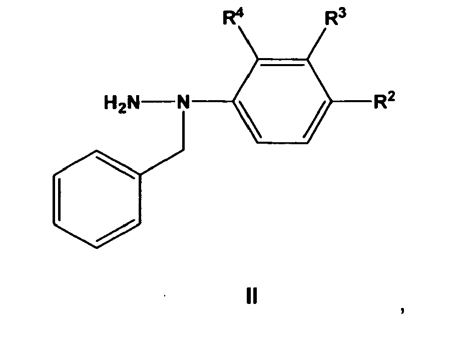 wherein R², R³ and R⁴ have the meaning as defined above, with at least one compound of general formula (III), 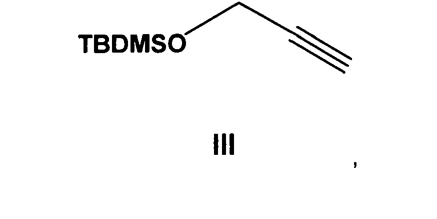 in the presence of a catalyst, preferably Ti(NEt₂)₄/2,6-tert-butyl-4-methylphenol or ZnCl₂, preferably in a reaction medium,e.g. toluene, for prolonged time, e.g. for the duration of 12 to 72 h at an elevated temperature, preferably from 50 to 150°C, more preferably from 80 to 120°C, to yield at least one compound of general formula (IV) 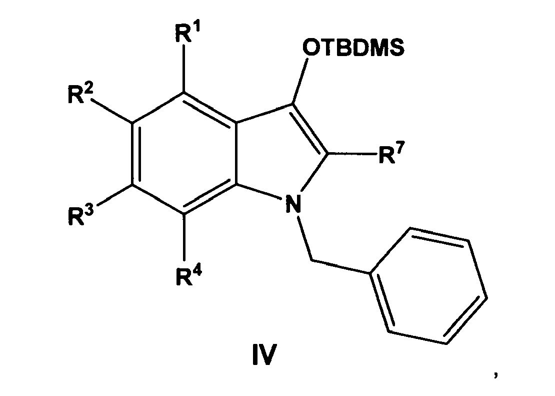 wherein R¹, R², R³ and R⁴ have the meaning as defined above, which is either reacted with at least one compound of general formula (V)

**R⁵R⁶NCH₂CH₂Cl** **V**

wherein R⁵ and R⁶ have the meaning as defined above or TBAF, in a reaction medium, e.g. THF, preferably in the presence of a base, preferably KOH, for prolonged time, e.g. the duration of 3 to 12 h at an elevated temperature, preferably from 50 to 150°C, more preferably from 60 to 110°C, to yield at least one compound of general formula (VI) 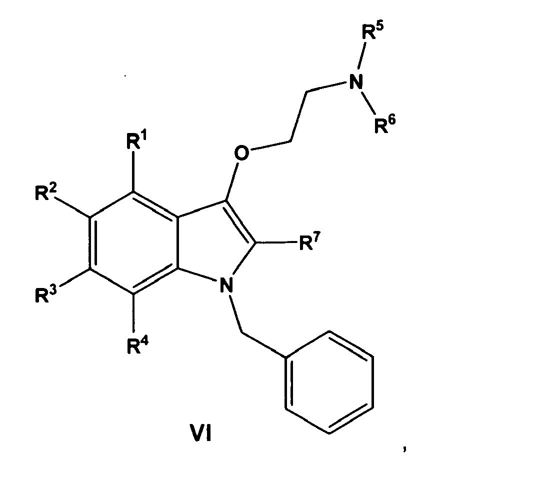 wherein R¹, R², R³, R⁴, R⁵, R⁶ and R⁷ have the meaning as defined above, which is reacted with a deprotecting reagent such as NH₃/Na in a preferably polar aprotic reaction medium, e.g. THF, for prolonged time, preferably for the duration of 1 to 6 h, more preferably 1 to 4 h, at a temperature from e.g. -120 to -10°C, preferably -100 to -50°C, to yield at least one compound of general formula (VII) 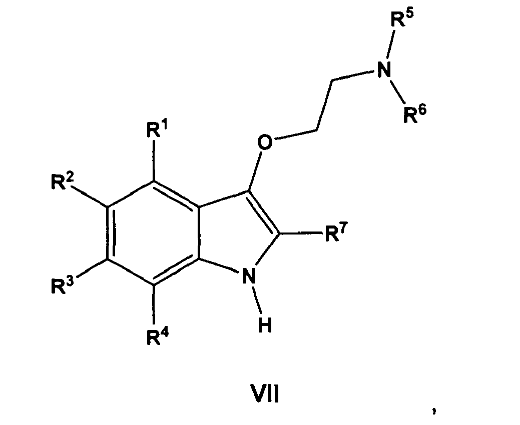 wherein R¹, R², R³, R⁴, R⁵, R⁶ and R⁷ have the meaning as defined above.

The general process for the preparation of compounds of general formula (I) as described above, is also depicted in general scheme I:

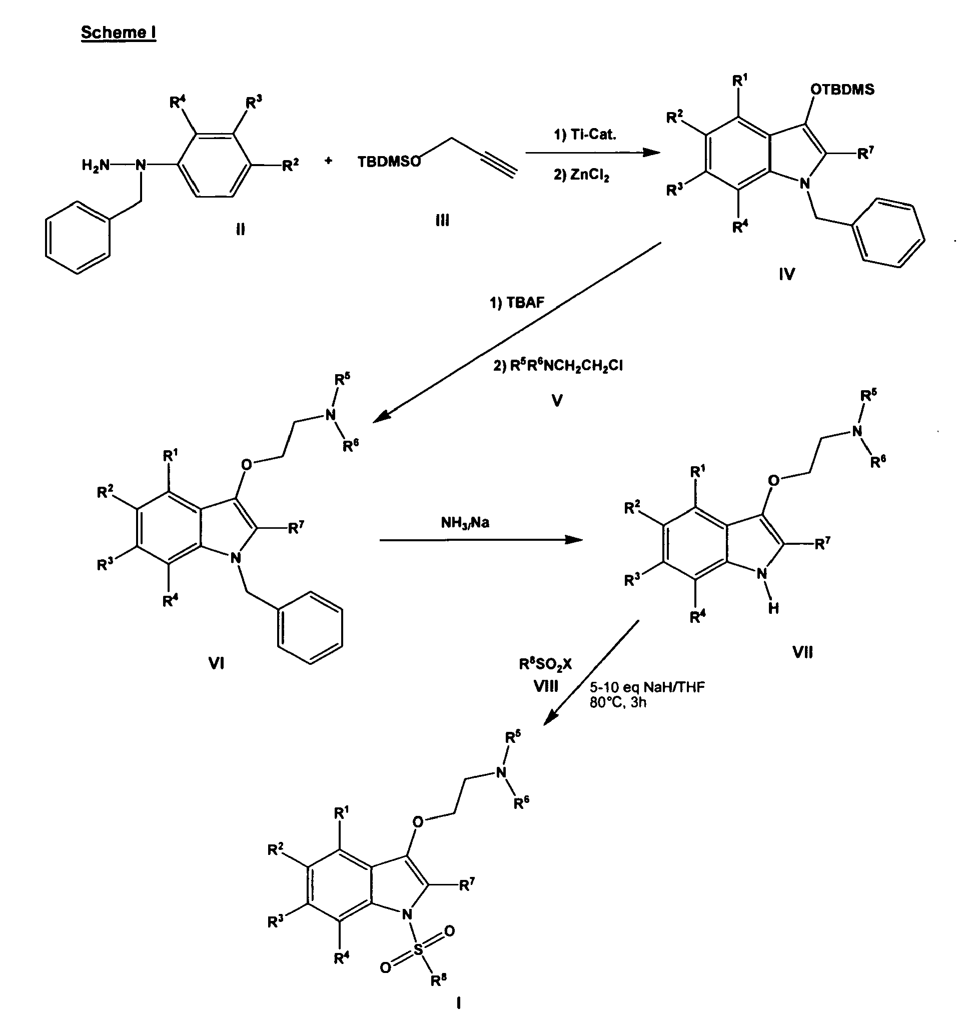

The compounds of general formula (I), including compounds of general formulae (Ia), (Ib), (Ic), (Id), (Ie), (If), (Ig) and (Ih) given above may be purified and/or isolated according to methods well known to those skilled in the art. Preferably, the compounds of general formula (I), including compounds of general formulae (Ia), (Ib), (Ic), (Id), (Ie), (If), (Ig) and (Ih) may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

During some synthetic reactions described above or while preparing the compounds of general formula (I), including compounds of general formulae (Ia), (Ib), (Ic), (Id), (Ie), (If), (Ig) and (Ih), the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted indole sulfonamide compounds of general formula (I), including compounds of general formulae (Ia), (Ib), (Ic), (Id), (Ie), (If), (Ig) and (Ih) are obtained in form of a mixture of stereoisomers, particularly enantiomers or diastereomers, said mixtures may be separated by standard procedures known to those skilled in the art, e.g. chromatographic methods or crystallization with chiral reagents.

The substituted indole sulfonamide compounds of general formula (I), including compounds of general formulae (Ia), (Ib), (Ic), (Id), (Ie), (If), (Ig) and (Ih) and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above. Suitable inorganic acids include but are not limited to hydrochloric acid, hydrobromic acid, phosphoric acid, oxalic acid, sulfuric acid, nitric acid, suitable organic acids include but are not limited to citric acid, maleic acid, fumaric acid, tartaric acid, or derivatives thereof, p-toluenesulfonic acid, methanesulfonic acid or camphersulfonic acid.

The term "salt" is to be understood as meaning any form of the substituted indole sulfonamide compounds in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the substituted indole sulfonamide compounds of general formula (I), including compounds of general formulae (Ia), (Ib), (Ic), (Id), (Ie), (If), (Ig) and (Ih) and in each case of corresponding stereoisomers may also be obtained by standard procedures known to those skilled in the art.

The term "solvate" according to this invention is to be understood as meaning any form of the substituted indole sulfonamide compounds in which they have attached to it via non-covalent binding another molecule (most likely a polar solvent) especially including hydrates and alcoholates, e.g. methanolate.

A further aspect of the present invention relates to a medicament comprising at least one substituted indole sulfonamide compound of general formula (I), including compounds of general formulae (Ia), (Ib), (Ic), (Id), (Ie), (If), (Ig) and (Ih) given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one physiologically acceptable auxiliary agent.

Said medicament is particularly suitable for 5-HT₆ receptor regulation and therefore for the prophylaxis and/or treatment of a disorder or a disease that is least partially mediated via 5-HT₆ receptors.

In a preferred embodiment of the present invention said medicament is suitable for the prophylaxis and/or treatment of food intake disorders, preferably for the regulation of appetite, for the maintenance, increase or reduction of body weight, for the prophylaxis and/or treatment of obesity, for the prophylaxis and/or treatment of bulimia, for the prophylaxis and/or treatment of anorexia, for the prophylaxis and/or treatment of cachexia, for the prophylaxis and/or treatment of type II diabetes (non-insulin dependent diabetes mellitus); for the prophylaxis and/or treatment of cognitive disorders, preferably memory disorders; or for improvement of cognition (for cognitive enhancement).

In another preferred embodiment of the present invention, said medicament is suitable for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment of panic attacks; for the prophylaxis and/or treatment of depression; for the prophylaxis and/or treatment of bipolar disorders; for improvement of cognition (for cognitive enhancement); for the prophylaxis and/or treatment of senile dementia; for the prophylaxis and/or treatment of psychosis; for the prophylaxis and/or treatment of neurodegenerative disorders; preferably selected from the group consisting of Morbus Alzheimer, Morbus Parkinson, Morbus Huntington and Multiple Sclerosis; for the prophylaxis and/or treatment of schizophrenia or for the prophylaxis and/or treatment of hyperactivity disorder (ADHD, attention deficit, hyperactivity disorder).

The medicament obtained according to the present invention is particularly suitable for the administration to mammals, including man. The drug can preferably be administered to all age groups, namely, children, adolescents and adults.

Another aspect of the present invention is the use of at least one indole sulfonamide derivative of general formula (I), including compounds of general formulae (Ia), (Ib), (Ic), (Id), (Ie), (If), (Ig) and (Ih) given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate, for the manufacture of a drug for regulating the 5-HT₆ receptor, for the prophylaxis and/or treatment of food intake disorders, preferably for the regulation of appetite, for the maintenance, increase or reduction of body weight, for the prophylaxis and/or treatment of obesity, for the prophylaxis and/or treatment of bulimia, for the prophylaxis and/or treatment of anorexia, for the prophylaxis and/or treatment of cachexia, for the prophylaxis and/or treatment of type II diabetes (non-insulin dependent diabetes mellitus); for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment cognitive disorders, preferably memory disorders; for improvement of cognition (for cognitive enhancement); for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia or for the prophylaxis and/or treatment hyperactivity disorder (ADHD, attention deficit, hyperactivity disorder).

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parenterally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective indole sulfonamide compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more substituted indole sulfonamide compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from 0.01 to 4000 mg, preferably 0.1 to 2000 mg, more preferably 0.5 to 1000 of active substance to be administered during one or several intakes per day.

In the following methods for determining the pharmacological activity of the substituted indole sulfonamide compounds are described.

### PHARMACOLOGICAL METHODS:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

The present invention is illustrated below with the aid of examples. These illustrations are given solely by way of example and do not limit the general spirit of the present invention.

### Examples

### Abbreviations:

- H: hour
- °C: °Celsius
- MI: milliliter
- Min: minute
- M: molar (concentration)
- TBDMSO: tert-butyl dimethylsilyloxy
- Ti(NEt₂)₄: tetrakis(diethylamino)titanium
- TBAF: tetrabutyl-ammonium fluoride
- THF: tetrahydro furane
- DMF: dimethyl formamide
- DMAc: dimethyl acetamide
- NMP: N-methyl-2-pyrrolidone
- ZnCl₂: zinc(II) chloride
- Et₂NCH₂CH₂Cl: 2-chloro-N,N-diethylethanamine
- KOH: potassium hydroxide
- NaH: sodium hydride
- Et₃N: triethylamine
- EtOAc: ethyl acetate
- Na₂SO₄: sodium sulfate
- CHCl₃: chloroform
- CH₂Cl₂: dichloromethane
- MgSO₄: magnesium sulphate
- MeOH: methanol

The compounds of general formula (III) can be prepared as indicated in scheme II indicated below.

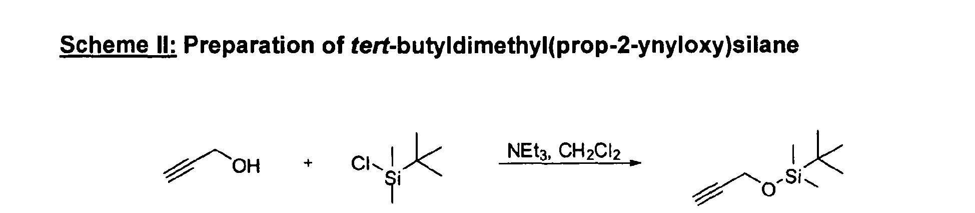

A solution of tert-butyldimethylsilyl chloride (3.7 g, 23.5 mmol) in 5 ml CH₂Cl₂ was added slowly to a solution of propargylic alcohol (1.16 ml, 1.12 g, 20 mmol) and Et₃N (3.78 ml, 2.75 g, 27.2 mmol) in 25 ml of CH₂Cl₂ at 0°C. The mixture was warmed to room temperature, stirred for 4 h, and treated with 30 ml of water. After the organic layer was separated, the aqueous layer was extracted with 1:1 hexane-EtOAc (2 x 30 ml). The combined organic layers were washed with brine (25 ml) and dried (Na₂SO₄). Removal of the solvent under reduced pressure followed by flash chromatography of the residue on silica gel (hexane) gave 3.85 ml (3.13 g, 18.4 mmol, 92 %) of *tert*-butyldimethyl(prop-2-ynyloxy)silane as a colorless liquid.
¹H NMR (300.13 MHz, CDCl₃): δ = 4.31 (d, 2H, ⁴J_{1,3} = 2.4 Hz, H-3); 2.39 (t, 1 H, ⁴J_{1,3} = 2.4 Hz, H-1); 0.92 (s, 9H, H-5); 0.13 (s, 6H, H-4) ppm. ¹³C NMR (75.5 MHz, CDCl₃): δ = 82.6, 73.0 (C-1,2); 51.7 (C-3); 26.0 (C-5); 18.5 (C-6); -5.0 (C-4) ppm. MS (EI, 70 eV): m/z (relative intensity): 170 (4) [M⁺], 155 (6), 137 (1), 125 (2), 113 (100), 99 (6), 83 (100), 75 (25), 59 (16), 45 (19), 43 (14). HRMS (EI): calcd. for C₉H₁₈OSi: 170.1121; found: 170.1115.

The compounds of general formulae (V) and (VIIII) are commercially available or may also be prepared according to standard methods known in the prior art.

### Procedure for synthesis of N,N-diethyl-2-(2-methyl-1-(naphthalen-2-ylsulfonyl)-1 H-indol-3-yloxy)ethanamine (Example compound 2)

*N*,*N*-diethyl-2-(2-methyl-1-(naphthalen-2-ylsulfonyl)-1H-indol-3-yloxy)ethanamine (Example compound 2) can be prepared according to the general scheme III depicted below.

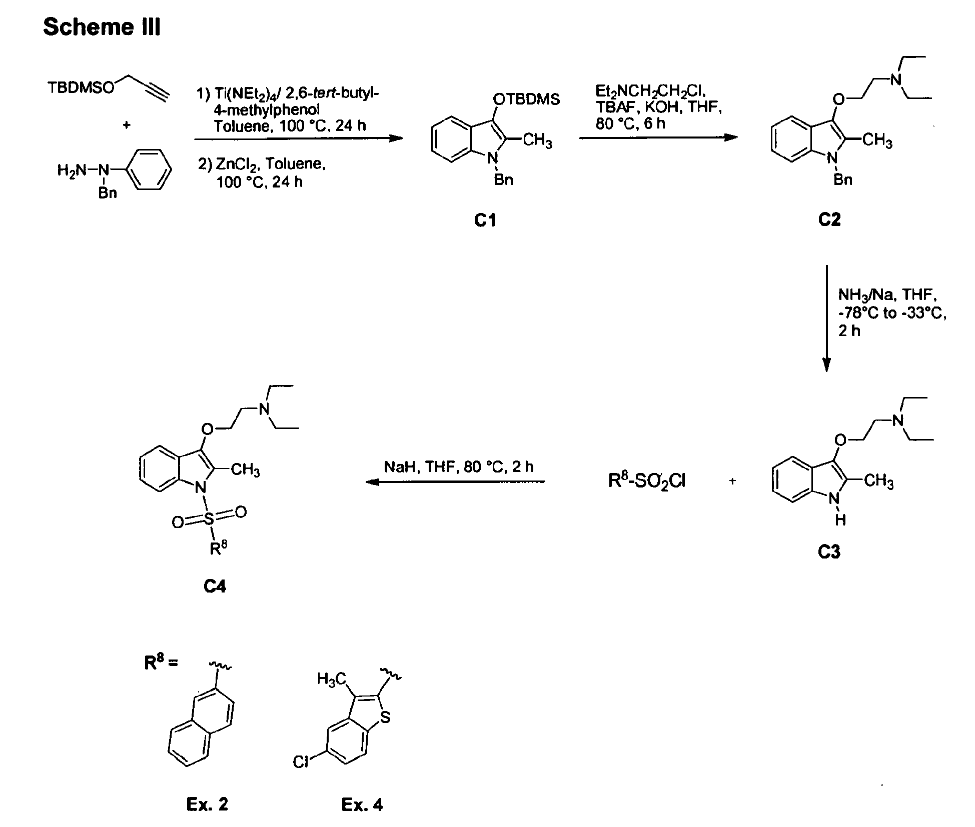

Compound C1 corresponds to a compound with general formula IV as indicated above in scheme I, with R¹, R², R³ and R⁴ each representing a hydrogen atom and R⁷ being a -CH₃ group.

Compound C2 corresponds to a compound with general formula VI as indicated above in scheme I, with R¹, R², R³ and R⁴ each representing a hydrogen atom, R⁵ and R⁶ each representing a -CH₂-CH₃ group and R⁷ being a -CH₃ group.

Compound C3 corresponds to a compound with general formula VII as indicated above in scheme I, with R¹, R², R³ and R⁴ each representing a hydrogen atom, R⁵ and R⁶ each representing a -CH₂-CH₃ group and R⁷ being a -CH₃ group.

Depending on the substituent R⁸, either Example compound 2 or 4 was obtained.

The preparation of the intermediate compounds C1, C2 and C3 is illustrated in scheme III given above and described below.

### Preparation of Compound C1:

### 1-Benzyl-3-(tert-buty)dimethylsoxyloxy)-2-methy)-1H-indole

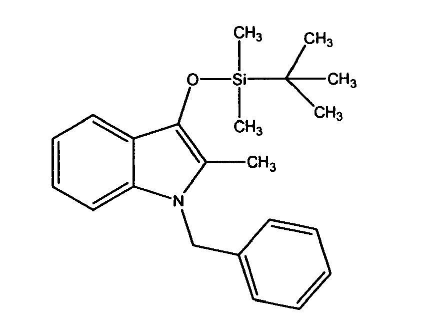

In a round bottom flask under an argon atmosphere the ligand 2,6-*tert*-butyl-4-methylphenol (60.4 mg, 0.27 mmol) was dissolved in 10 ml dry toluene. To this solution *N*-benzyl-*N*-phenylhydrazine (0.52 ml, 3.56 mmol), *tert*-butyldimethyl(prop-2-ynyloxy)silane (0.57 ml, 2.74 mmol) and Ti(NEt₂)₄ (50µl, 0.14 mmol) were added. The reaction mixture was heated at 100 °C for 24 h. In the following the round bottom flask was opened under argon to add ZnCl₂ (1.12 g, 8.2 mmol). The reaction mixture was again heated at 100 °C for 24 h. When the mixture had risen to room temperature the solution was decanted and the black residue of the ZnCl₂ was washed with toluene and ethylacetate. After removal of the combined solvents *in vacuo,* the desired indole product was isolated by column chromatography in hexane/ethylacetate (10:1). Isolated yield; 50 % (529 mg, 1.37 mmol), light yellow crystals (mp = 69 °C).
¹H NMR (300 MHz, CDCl₃): δ = 7.45-7.40 (m, 1 H, H-4); 7.27-7.21 (m, 3H, m-, p-Ph); 7.10 (m, 1 H, H-7); 6.93 (m, 2H, H-6, H-5); 6.86-6.87 (m, 2H, o-Ph); 5.22 (s, 2H, CH₂Ph, H-10); 2.20 (s, 3H, H-11); 0.83 (s, 12H, H-14); 0.15 (s, 6H, H-12) ppm. ¹³C NMR (75 MHz, CDCl₃): δ = 136.4 (i-Ph); 135.6 (C-3); 133.6 (C-8); 128.9 (m-Ph); 128.4 (C-2); 127.8 (p-Ph); 125.8 (o-Ph); 121.7, 120.6 (C-9,5); 120.8 (C-6); 120.7 (C-7); 108.7 (C-7); 46.9 (C-10); 25.8 (C-14); 18.2 (C-13); 9.1 (C-11); -5.3 (C-12) ppm. MS (EI, 70 eV): *m*/*z* (relative intensity): 351 (100) [M⁺], 294 (27), 260 (13), 221 (25), 204 (12), 177 (8), 115 (15), 91 (90), 73 (71), 65 (8), 43 (6). HRMS (EI): calcd. for C₂₂H₂₉NOSi: 351.2013; found: 351.1999.

### Preparation of Compound C2:

### 2-(1-Benzyl-2-methyl-1 H-indole-3-yloxy)-N,N-diethylethanamine

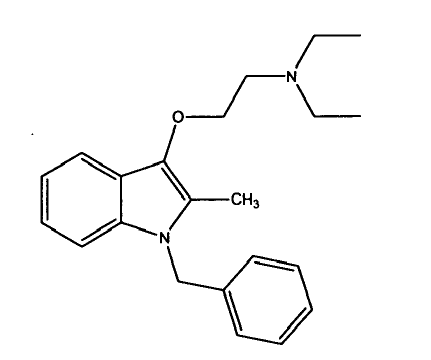

To powdered potassium hydroxide (84.2 mg, 1.5 mmol) in a round bottom flask under an argon atmosphere 15 ml dry THF and TBAF (2.75 ml of 1 M in THF, 2.75 mmol) was added. After the addition of the indole (**C1**) (529 mg, 1.37 mmol) and diethylami-noethylchloride (202.5 mg, 1.5 mmol) the mixture was stirred at 80 °C for 6 h. When the mixture had risen to room temperature, H₂O (15 ml) was added. Then the separated aqueous layer was extracted with CHCl₃ (3 x 20 ml). The organic layers were dried (Na₂SO₄) and the solvents were evaporated *in vacuo.* The residue was chromatographed on a silica gel column (eluent: CHCl₃/10% MeOH) to give a yellow oil of compound **C2** (286 mg, 0.85 mmol) in 62% yield.
¹H NMR (300 MHz, CDCl₃): δ = 7.62-7.59 (m, 1H, H-4); 7.26-7.16 (m, 4H, m-,p-Ph, H-7); 7.09-7.05 (m, 2H, H-6, H-5); 6.93-6.90 (m, 2H, o-Ph); 5.23 (s, 2H, CH₂Ph, H-10); 4.17 (t, J = 6.4 Hz, 2H, H-12); 2.90 (t, J = 6.4 Hz, 2H, H-13); 2.65 (q, J = 7.1 Hz, 4H, H-14); 2.28 (s, 3H, H-11); 1.06 (t, J = 7.1 Hz, 6H, H-15) ppm. ¹³C NMR (75 MHz, CDCl₃): δ = 137.9 (i-Ph); 135.1 (C-3); 133.7 (C-8); 128.6 (m-Ph); 127.1 (C-2); 125.8 (p-Ph); 124.6 (o-Ph); 121.1, 120.9 (C-9,5); 118.8 (C-6); 117.0 (C-7); 109.0 (C-7); 72.6 (C-12), 52.6 (C-13); 47.5 (C-14); 46.3 (C-10); 11.7 (C-15); 8.8 (C-11) ppm . MS (EI, 70 eV): *m*/*z* (relative intensity): 336 (6) [M⁺], 279 (2), 237 (7), 221 (4), 208 (2), 195 (58), 180 (6), 165 (9), 117 (5), 100 (100), 91 (36), 86 (12), 71 (5), 57 (7), 43 (16). HRMS (EI): calcd. for C₂₂H₂₈N₂O: 336.2196; found: 336.2193.

### Preparation of Compound C3:

### N,N-diethyl-2-(2-methyl-1 H-indole-3-yloxy)ethanamine

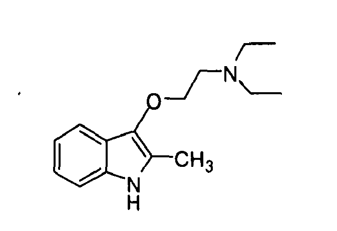

To a deep blue solution of Na (195 mg, 8.5 mmol) in NH₃ (ca. 20 ml) at -78 °C a solution of compound **C2** (286 mg, 0.85 mmol) in dry THF (4 ml) was added dropwise. The mixture was stirred at -33 °C for 2 h, quenched with solid NH₄Cl at -78 °C, allowed to warm to room temperature, and concentrated. The residue was diluted with H₂O and extracted with CHCI₃ (3 x 20 ml). The combined organic layers were dried over Na₂SO₄, filtered and the solvents were removed *in vacuo* to give a yellow oil. The crude material is used for the next reaction.
¹H NMR (300 MHz, CDCl₃): δ = 7.73 (br s, 1H, NH); 7.57-7.54 (dd, J = 6.9, J = 1.9, 1 H, H-4); 7.10 (dd, J = 6.9, J = 1.9,1 H, H-7); 7.12-7.03 (m, 2H, H-6, H-5); 4.16 (t, J = 6.5 Hz, 2H, H-11); 2.91 (t, J = 6.5 Hz, 2H, H-12); 2.67 (q, J = 7.1 Hz, 4H, H-13); 2.35 (s, 3H, H-10); 1.08 (t, J = 7.1 Hz, 6H, H-14) ppm. ¹³C NMR (75 MHz, CDCl₃): δ = 135.1 (C-3); 132.7 (C-8); 122.5 (C-2); 121.9 (C-9); 120.9 (C-5); 118.9 (C-6); 116.9 (C-7); 110.6 (C-5); 72.3 (C-11); 52.6 (C-12); 47.5 (C-13); 11.6 (C-14); 10.2 (C-10) ppm. MS (EI, 70 eV): m/z (relative intensity): 246 (1) [M⁺], 160 (1), 146 (11), 117 (5), 100 (100), 86 (40), 72 (11), 56 (7), 44 (18), 29 (6). HRMS (EI): calcd. for C₁₅H₂₂N₂O: 246.19870; found: 246.20062.

### Preparation of Example compound 2 (general formula Ig):

### N,N-diethyl-2-(2-methyl-1-(naphthalene-2-ylsulfonyl)-1 H-indole-3-yloxy)ethanamine

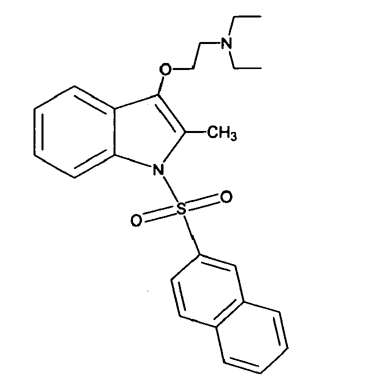

The aforementioned crude **C3** in dry THF (3 ml) was added dropwise to NaH (120 mg, 5 mmol, 65 % content in a mineral oil suspension that was washed with dry *n-*hexane three times before use) suspended in dry THF (5 ml) at room temperature under argon, and then the mixture was stirred for 10 min. A dry THF (3 ml) solution of 2-naphthaline-sulfonylchloride (340 mg, 1.5 mmol) was added to the mixture, and the resulting solution was stirred at 80 °C for 2 h. The reaction was quenched by adding aq. Na₂CO₃ (20 ml) and the mixture was extracted with CHCl₃ (3 x 20 ml). After drying with Na₂SO₄, removal of the solvent and chromatography of the crude material with CHCl₃/10% MeOH gave Example compound 2 as a brown oil (100.4 mg, 0.23 mmol) with 27 % yield for 2 steps.
¹H NMR (300 MHz, CDCl₃): δ = 8.37-8.36 (m, 1 H, H-23); 8.28-8.25 (d, J = 8.2, 1 H, H-17); 7.91-7.88 (m, 1H, H-16); 7.81-8.75 (m, 2H, H-21, H-19); 7.62-7.53 (m, 3H, H-4, H-20); 7.47-7.44 (m, 1 H, H-7); 7.12-7.03 (m, 2H, H-6, H-5); 4.02 (t, J = 6.3 Hz, 2H, H-11); 2.75 (t, J = 6.3 Hz, 2H, H-12); 2.58-2.51 (q, J = 7.1 Hz, 4H, H-13); 2.57 (s, 3H, H-10); 0.98 (t, J = 7.1 Hz, 6H, H-14) ppm. ¹³C NMR (75 MHz, CDCl₃): δ = 141.3 (C-18); 135.7 (C-15); 135.1 (C-3); 134.7 (C-22); 131.8 (C-8); 129.5 (C-17); 129.4 (C-19); 129.2 (C-21); 127.9 (C-23); 127.8 (C-20); 125.1 (C-2); 124.4 (C-9); 123.4 (C-5); 121.1 (C-6); 117.5 (C-7); 115.0 (C-7); 72.2 (C-11); 52.6 (C-12); 47.5 (C-13); 11.7 (C-14); 11.4 (C-10) ppm. MS (EI, 70 eV): m/z (relative intensity): 436 (1) [M⁺], 160 (1), 146 (11), 117 (5), 100 (100), 86 (40), 72 (11), 56 (7), 44 (18), 29 (6). HRMS (EI): calcd. for C₂₅H₂₈N₂O₃S: 436.19870; found: 436.20062.

### Preparation of Example compound 4 (general formula Id):

### 2-(1-(5-chloro-3-methylbenzo[b]thiophen-2-ylsulfonyl)-2-methyl-1H-indol-3-yloxy)-N,N-diethylethanamine

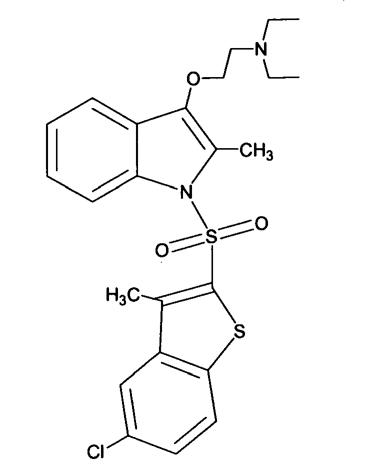

According to the procedure in the preparation of Example compound 2, 5-chloro-3-methylbenzo[b]thiophene-2-sulfonyl-chloride was employed. The product was first columned with CHCl₃/10% MeOH and a second chromatography with ethylacetate/10% MeOH gave Example compound 4 as a brown oil (62.6 mg,0.13 mmol) with 15 % yield over 2 steps.
¹H NMR (500.13 MHz, CDCl₃): δ = 7.71 (br d, 1H, ³*J*_{6,7} = 8.2 Hz, H-7); 7.61 (d, 1 H, ⁴*J*_{11,13} = 1.0 Hz, H-11); 7.51 (br d, 1H, ³*J*_{4,5} = 8.0 Hz, H-4); 7.48 (d, 1H, ³*J*_{13,14} = 8.5 Hz, H-14); 7.25-7.21 (m, 2H, H-6,13); 7.13 (ddd, 1H, ³*J*_{4,5} = 8.0 Hz, ³*J*_{5,6} = 6.9 Hz, ⁴*J*_{5,7} = 1.0 Hz, H-5); 4.13 (t, 2H, ³*J*_{17,18} = 6.5 Hz, H-17); 2.89 (t, 2H, ³*J*_{17,18} = 6.5 Hz, H-18); 2.67 (q, 4H, ³*J*_{19,20} = 7.3 Hz, H-19); 2.62 (s, 3H, Me₍₁₆₎); 2.49 (s, 3H, Me₍₁₅₎); 1.05 (t, 6H, ³*J*_{19,20} = 7.3 Hz, H-20) ppm. ¹³C NMR (125.8 MHz, CDCl₃): δ = 140.5, 138.9, 138.4, 137.6, 135.3, 133.7, 130.8, 127.8, 123.1 (C-2,3,3a,7a,9,10,10a,12,14a); 125.8 (C-13); 123.1 (C-14); 122.4 (C-6); 122.2 (C-11); 121.1 (C-5); 117.3 (C-4); 72.0 (C-17); 52.6 (C-18); 47.6 (C-19); 12.9 (C-16); 11.6 (C-20); 9.8 (C-15) ppm. MS (El, 70 eV): *m*/*z* (relative intensity): 490 (1) [M⁺], 245 (6), 214 (53), 180 (31), 100 (100), 86 (44), 72 (7), 56 (7), 44 (7). HRMS (EI): calcd. for C₂₄H₂₇ClN₂O₃S₂: 490.11461; found: 490.11321.

## Claims

1. Substituted indole sulfonamide compounds of general formula (I), 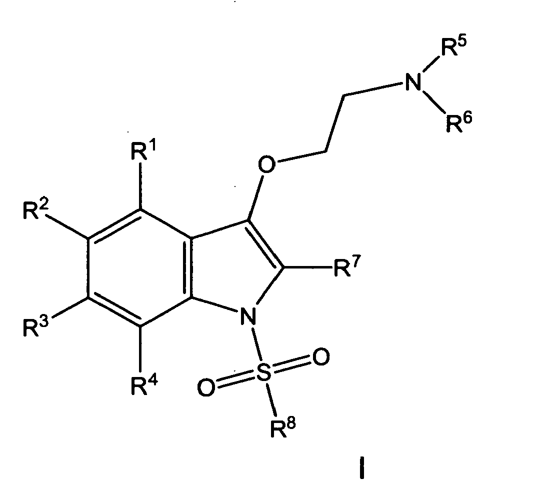 wherein
R¹, R², R³ and R⁴, independent from one another, each represents a hydrogen atom; a halogen atom; -NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁹; -S(=O)₂-R¹⁰; -OR¹¹; -SR¹²; -C(=O)-OR¹³; -NH-R¹⁶; -NR¹⁷R¹⁸; -C(=O)-NHR¹⁹; -C(=O)-NR²⁰R²¹; -S(=O)₂-NHR²²; -S(=O)₂-NR²³R²⁴; -O-C(=O)-R²⁵; -NH-C(=O)-R²⁶; -NR²⁷-C(=O)-R²⁸; -NH-C(=O)-O-R²⁹; -NR³⁰-C(=O)-O-R³¹; -S(=O)₂-O-R³²;
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted saturated, unsaturated or aromatic mono- or bicyclic ring system;
or
an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted saturated or unsaturated or aromatic mono- or bicyclic ring system;
R⁵ and R⁶, independent from one another, each represents a hydrogen atom; or a linear or branched, saturated or unsaturated, unsubstituted or at least mono-substituted aliphatic radical;
or
R⁵ and R⁶ together with the bridging nitrogen atom form a saturated or unsaturated, unsubstituted or at least mono-substituted, optionally at least one additional heteroatom as ring member containing heterocycloaliphatic radical, which may be condensed with an unsubstituted or at least mono-substituted saturated, unsaturated or aromatic mono- or bicyclic ring system;
or
R⁵ and R⁶ together with the bridging nitrogen atom form an unsubstituted or at least mono-substituted, optionally at least one additional heteroatom as ring member containing heteroaryl radical, which may be condensed with an unsubstituted or at least mono-substituted saturated, unsaturated or aromatic mono-or bicyclic ring system;
R⁷ represents a hydrogen atom; or a linear or branched, saturated or unsaturated, unsubstituted or at least mono-substituted aliphatic radical;
R⁸ represents a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted saturated, unsaturated or aromatic mono- or bicyclic ring system;
an unsubstituted or at least mono-substituted phenyl radical, which is bonded via an unsubstituted or at least mono-substituted, linear or branched alkylene group;
an unsubstituted or at least mono-substituted 10- or 14-membered aryl radical, which may be bonded via a linear or branched, unsubstituted or at least mono-substituted alkylene, alkenylene or alkinylene group and/or which may be condensed with an unsubstituted or at least mono-substituted, saturated, unsaturated or aromatic mono- or bicyclic ring system;
an unsubstituted or at least mono-substituted 5- or 6-membered heteroaryl radical, which may be bonded via an unsubstituted or at least mono-substituted, linear or branched alkylene, alkenylene or alkinylene group and/or which may be condensed with an unsubstituted or at least mono-substituted, saturated, unsaturated or aromatic mono- or bicyclic ring system;
or an unsubstituted or at least mono-substituted ring system selected from the group consisting of: 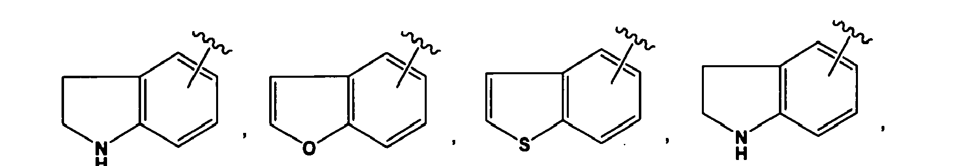 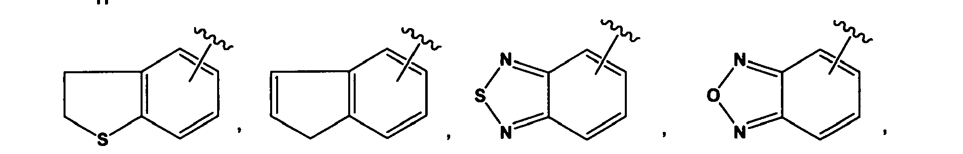 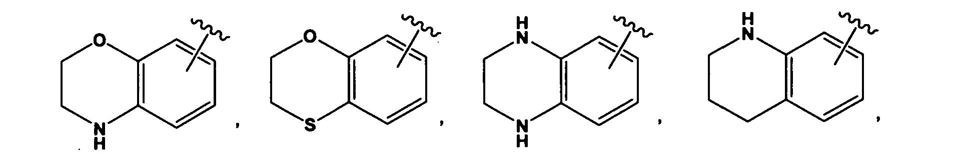 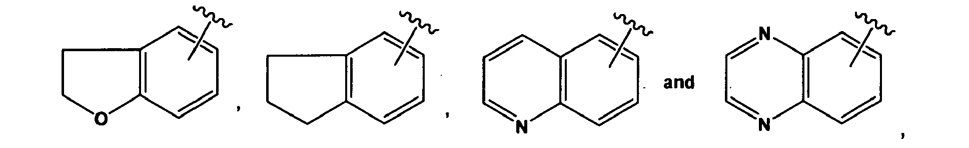 whereby these afore depicted moieties are bonded to the general formula (I) via their aromatic part;
R⁹, R¹⁰, R¹¹, R¹², R¹³, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹ and R³², independent from one another, each represents a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted saturated, unsaturated or aromatic mono- or bicyclic ring system;
or
an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted saturated, unsaturated or aromatic mono- or bicyclic ring system;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

2. Compounds according to claim 1,
wherein
R¹, R², R³ and R⁴, independent from one another, each represents a hydrogen atom; F; Cl; Br; I; -NO₂, -NH₂; -SH; -OH; -CN; -C(=O)-OH; -C(=O)-H; -S(=O)₂-OH; -C(=O)-NH₂; -S(=O)₂-NH₂; -C(=O)-R⁹; -S(=O)₂-R¹⁰; -OR¹¹; -SR¹²; -C(=O)-OR¹³; -NH-R¹⁶; -NR¹⁷R¹⁸; -C(=O)-NHR¹⁹; -C(=O)-NR²⁰R²¹; -S(=O)₂-NHR²²; -S(=O)₂-NR²³R²⁴; -O-C(=O)-R²⁵; -NH-C(=O)-R²⁶; -NR²⁷-C(=O)-R²⁸; -NH-C(=O)-O-R²⁹; -NR³⁰-C(=O)-O-R³¹; -S(=O)₂-O-R³²;
a linear or branched, unsubstituted or at least mono-substituted C₁₋₁₀ alkyl radical, C₂₋₁₀ alkenyl radical or C₂₋₁₀ alkinyl radical;
an unsubstituted or at least mono-substituted C₃₋₉ (hetero)cycloalkyl radical or C₄₋₉ (hetero)cycloalkenyl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
or
an unsubstituted or at least mono-substituted 6-, 10- or 14-membered mono-, bi- or tricyclic aryl or a 5-, 6-, 8-, 9-, 10-, 11-, 12-, 13- or 14-membered mono-, bi- or tricyclic heteroaryl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
R⁵ and R⁶, independent from one another, each represents a hydrogen atom; or a linear or branched, unsubstituted or at least mono-substituted C₁₋₁₀ alkyl radical, C₂-₁₀ alkenyl radical or C₂₋₁₀ alkinyl radical;
or
R⁵ and R⁶ together with the bridging nitrogen atom form an optionally at least one additional heteroatom as a ring member containing C₃₋₉ heterocycloalkyl radical or C₄₋₉ heterocycloalkenyl radical, which may be condensed with an unsubstituted or at least mono-substituted saturated, unsaturated or aromatic mono- or bicyclic ring system;
or
R⁵ and R⁶ together with the bridging nitrogen atom form an unsubstituted or at least mono-substituted 5-, 6-, 8-, 9-, 10-, 11-, 12-, 13- or 14-membered mono-, bi- or tricyclic heteroaryl radical, which may be condensed with an unsubstituted or at least mono-substituted saturated, unsaturated or aromatic mono- or bicyclic ring system;
R⁷ represents a hydrogen atom; or a linear or branched, unsubstituted or at least mono-substituted C₁₋₁₀ alkyl radical, C₂₋₁₀ alkenyl radical or C₂₋₁₀ alkinyl radical;
R⁸ represents an unsubstituted or at least mono-substituted C₃₋₉ (hetero)cycloalkyl radical or C₄₋₉ (hetero)cycloalkenyl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
an unsubstituted or at least mono-substituted phenyl radical, which is bonded via a linear or branched, unsubstituted or at least mono-substituted C₁₋₆ alkylene, C₂₋₆ alkenylene or C₂₋₆ alkinylene group;
an unsubstituted or at least mono-substituted, 10- or 14-membered bi- or tricyclic aryl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted, saturated, unsaturated or aromatic mono- or bicyclic ring system;
an unsubstituted or at least mono-substituted 5- or 6-membered monocyclic heteroaryl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted, saturated, unsaturated or aromatic mono- or bicyclic ring system;
or
an unsubstituted or at least mono-substituted ring system selected from the group consisting of: 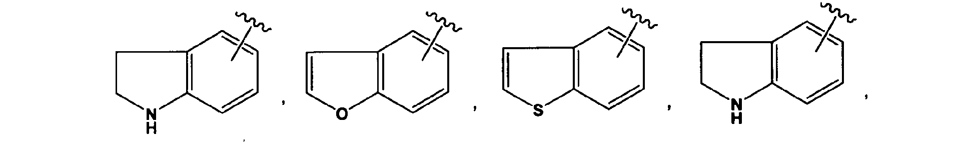 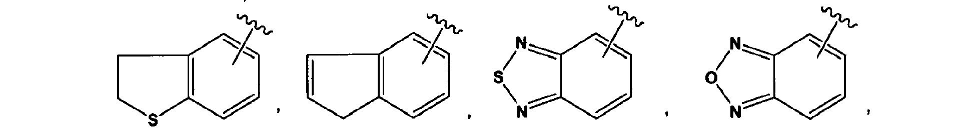 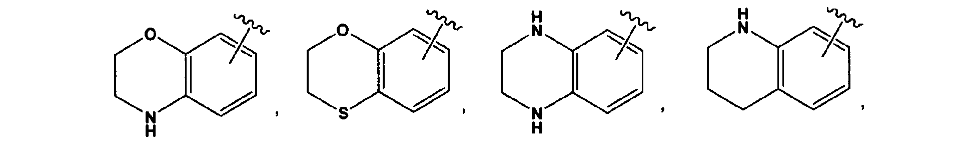 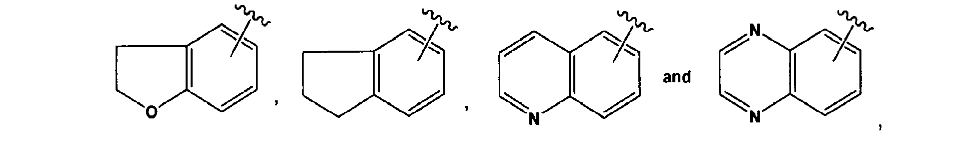 whereby these afore depicted ring systems are bonded to the general formula (I) via their aromatic phenyl part;
R⁹, R¹⁰, R¹¹, R¹², R¹³, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹ and R³², independent from one another, each represents a linear or branched, unsubstituted or at least mono-substituted C₁₋₁₀ alkyl radical, C₂₋₁₀ alkenyl radical or C₂₋₁₀ alkinyl radical;
an unsubstituted or at least mono-substituted C₃₋₉ (hetero)cycloalkyl or C₄₋₉ (hetero)cycloalkenyl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
or
an unsubstituted or at least mono-substituted 6-, 10- or 14-membered mono-, bi- or tricyclic aryl or a 5-, 6-, 8-, 9-, 10-, 11-, 12-, 13- or 14-membered mono-, bi- or tricyclic heteroaryl radical, which may be bonded via a linear or branched, unsubstituted or at least mono-substituted C₁₋₆ alkylene, C₂₋₆ alkenylene or C₂₋₆ alkinylene group and/or which may be condensed with an unsubstituted or at least mono-substituted saturated, unsaturated or aromatic mono- or bicyclic ring system;
whereby
the aforementioned C₁₋₁₀ alkyl radicals, C₂₋₁₀ alkenyl radicals or C₂₋₁₀ alkinyl radicals may in each case be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; whereby in each occurrence C₁₋₁₀-alkyl, C₂₋₁₀ alkenyl or C₂₋₁₀ alkinyl may be linear or branched;
the aforementioned C₃₋₉ (hetero)cycloalkyl or C₄₋₉ (hetero)cycloalkenyl radicals may in each case be unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenethyl, phenoxy and benzyl, whereby in each occurrence C₁₋₅-alkyl may be linear or branched and whereby the cyclic part(s) of said substituent(s) is unsubstituted or optionally substituted with 1, 2 or 3 substituent(s) independently selected from the group consisting of methyl, ethyl, n-propyl, isopropyl, methoxy, ethoxy, F, Cl, Br, I -CN, -CF₃, -OCF₃, -SCF₃, -OH, -SH, -NH₂ and -NO₂;
the aforementioned C₃₋₉ heterocycloalkyl or C₄₋₉ heterocycloalkenyl radicals may optionally contain 1 or 2 additional heteroatom(s) independently selected from the group consisting of nitrogen, oxygen and sulfur as ring member(s);
the aforementioned C₃₋₉ cycloalkyl and C₄₋₉ cycloalkenyl radicals in each case may optionally contain 1, 2 or 3 heteroatom(s) independently selected from the group consisting of nitrogen, oxygen and sulfur as ring member(s);
the aforementioned C₁₋₆ alkylene, C₂₋₆ alkenylene or C₂₋₆ alkinylene groups are in each case unsubstituted or optionally substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
the rings of the aforementioned ring systems are in each case independently of one another 5-, 6-, 7-, 8-, 9,- or 10-membered and may in each case independently of one another optionally contain 1, 2 or 3 heteroatom(s) independently selected from the group consisting of nitrogen, oxygen and sulphur;
and the rings of the aforementioned ring systems are in each case unsubstituted or optionally substituted with 1, 2, 3, 4 or 5 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenethyl, phenoxy and benzyl, whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, I, -CN, -CF₃, -OCF₃, -SCF₃, -OH, -SH, -NH₂ and -NO₂;
the 6-, 10- or 14-membered mono-, bi- or tricyclic aryl or 5-, 6-, 8-, 9-, 10-, 11-, 12-, 13- or 14-membered mono-, bi- or tricyclic heteroaryl radicals are unsubstituted or optionally substituted with 1, 2, 3, 4 or 5 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenethyl, phenoxy and benzyl, whereby in each occurrence C₁₋₅-alkyl may be linear or branched and whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, I, -CN, -CF₃, -OCF₃, -SCF₃, -OH, -SH, -NH₂ and -NO₂;
and the aforementioned 5-, 6-, 8-, 9-, 10-, 11-, 12-, 13- or 14-membered mono-, bi- or tricyclic heteroaryl radicals in each case contain 1, 2, 3 or 4 heteroatom(s) independently selected from the group consisting of nitrogen, oxygen and sulfur as ring member(s);
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

3. Compounds according to one or more of claims 1 to 2,
wherein
R¹, R², R³ and R⁴, independent from one another, each represents a hydrogen atom; F; Cl; Br; I; -NO₂; -SH; -OH; -CN; -OCF₃; -SCF₃; -NH₂; -O-CH₃; -O-C₂H₅; -S-CH₃; -S-C₂H₅; -NH-CH₃; -N(-CH₃)₂; -C(=O)-OH; -C(=O)-H; -S(=O)₂-OH; -C(=O)-NH₂; -S(=O)₂-NH₂;
a radical selected from the group consisting of methyl, ethyl, n-propyl, isopropyl, n-butyl, sec-butyl, isobutyl, tert-butyl, n-pentyl, neo-pentyl, n-hexyl, vinyl, allyl and ethinyl, which is unsubstituted or optionally substituted with 1, 2, 3, 4, or 5 substituents independently selected from the group consisting of F, Cl, Br, I, -OH, -SH, NH₂, -O-CH₃, -O-C₂H₅, -S-CH₃, -NH-CH₃ and -N(-CH₃)₂;
a (hetero)cycloaliphatic radical selected from the group consisting of cyclopropyl, cyclobutyl, cyclopentyl, cyclohexyl, cycloheptyl, imidazolidinyl, aziridinyl, azetidinyl, pyrrolidinyl, piperidinyl, morpholinyl, thiomorpholinyl, piperazinyl and pyrazolidinyl, which is unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, phenyl, benzyl, phenethyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂ and -NH-CH₃;
or
an aryl or heteroaryl radical selected from the group consisting of phenyl, naphthyl, thienyl (thiophenyl), pyrrolyl, imidazolyl, pyrazolyl, pyridinyl, pyridazinyl, pyrimidinyl, pyrazinyl, quinolinyl, isoquinolinyl and imidazo[2,1-b]thiazolyl, which is unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, phenyl, benzyl, phenoxy, phenethyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂ and -NH-CH₃;
R⁵ and R⁶, independent from one another, each represents a hydrogen atom or a radical selected from the group consisting of methyl, ethyl, n-propyl, isopropyl, n-butyl, sec-butyl, isobutyl, tert-butyl, n-pentyl, neo-pentyl, n-hexyl, vinyl, allyl and ethinyl, which is unsubstituted or optionally substituted with 1, 2, 3, 4, or 5 substituent(s) independently selected from the group consisting of F, Cl, Br, I, - OH and -SH;
or
R⁵ and R⁶ together with the bridging nitrogen atom form a ring system selected from the group consisting of: 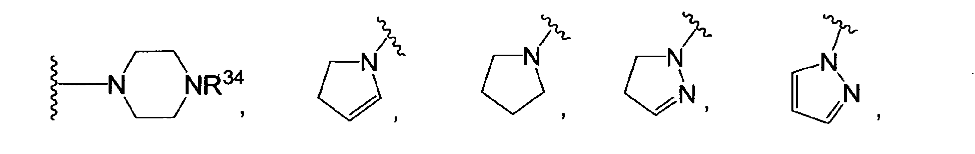 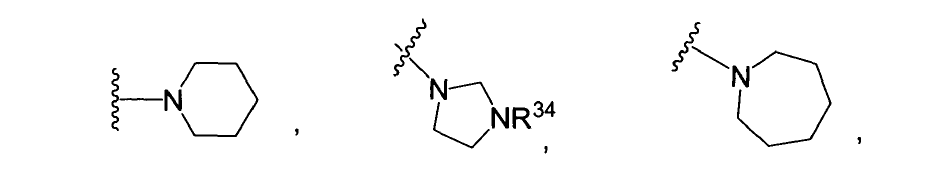 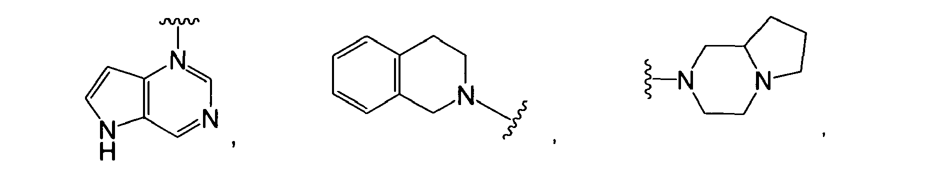 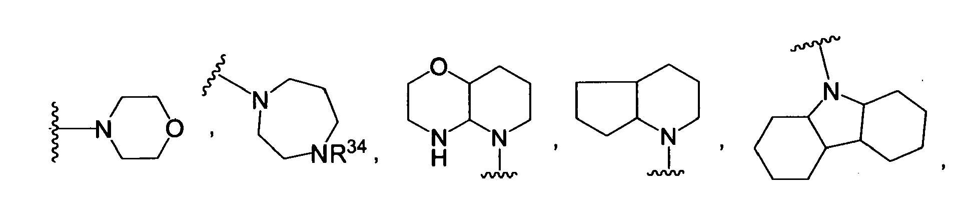 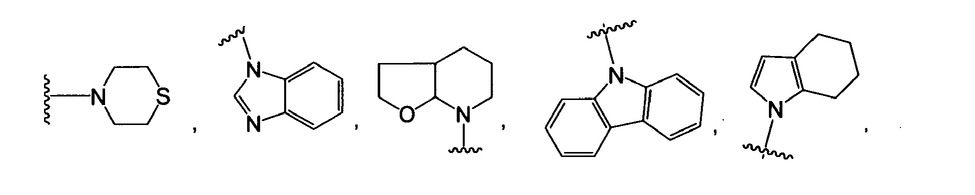 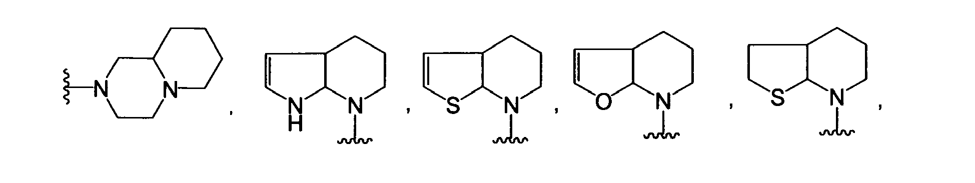 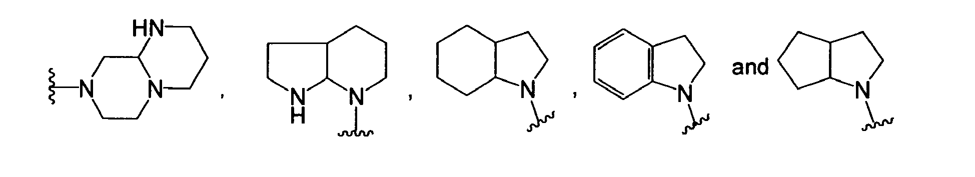 which is unsubstituted or optionally substituted in any suitable position with 1, 2, 3, 4 or 5 substituent(s) independently selected from the group consisting of oxo (=O), methyl, ethyl, n-propyl, isopropyl, n-butyl, tert-butyl, sec-butyl, phenyl, phenethyl, benzyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃, whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂;
R³⁴ represents a hydrogen atom; methyl; ethyl; n-propyl; isopropyl; phenyl; benzyl; phenethyl; -CF₃; -CHF₂; -CH₂F; -C(=O)-CH₃; -C(=O)-C₂H₅; -C(=O)-CH₂-CH₂-CH₃; -C(=O)-NH-CH₃ or a -C(=O)-N(CH₃)₂ moiety;
R⁷ represents a hydrogen atom or a methyl, ethyl, n-propyl, isopropyl, n-butyl, sec-butyl, isobutyl or tert-butyl moiety, which is unsubstituted or optionally substituted with 1, 2, 3, 4 or 5 substituent(s) independently selected from the group consisting of F, Cl, Br, I, -CN, -NH₂, -OH and -SH;
R⁸ represents a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, azepanyl (1,2,3,6)-tetrahydropyridinyl, (1,2,3,4)-tetrahydropyridinyl, (1,2,5,6)-tetrahydropyridinyl, hexahydropyrimidinyl, (5,6)-dihydro-4H-pyrimidinyl, indolinyl, isoindolinyl, (1,2,3,4)-tetrahydroquinolinyl, (1,2,3,4)-tetrahydroisoquinolinyl and octahydro-cyclopenta[c]pyrrolyl, which is unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, phenyl, benzyl, phenethyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃, whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂;
or a ring system selected from the group consisting of: 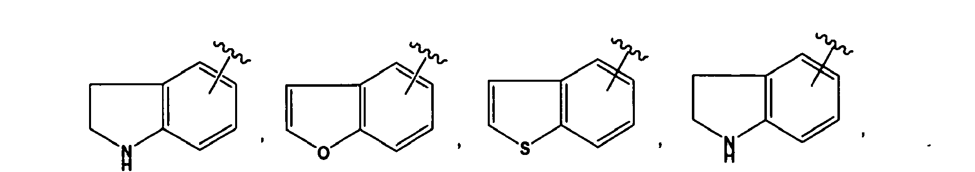 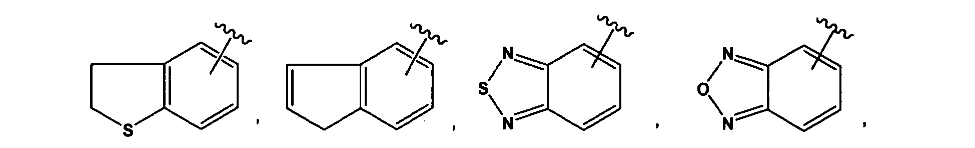 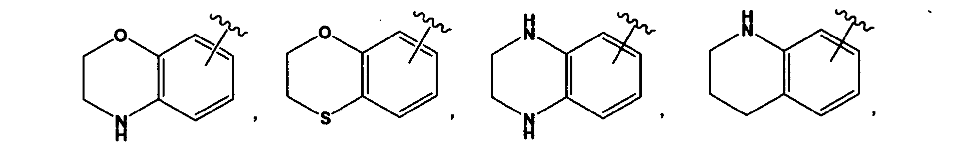 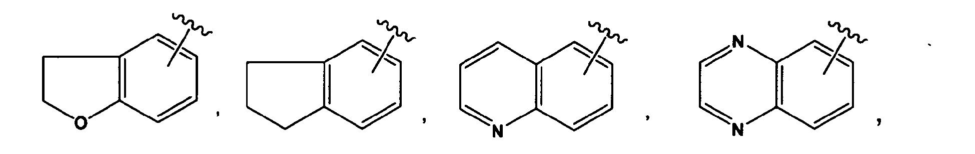 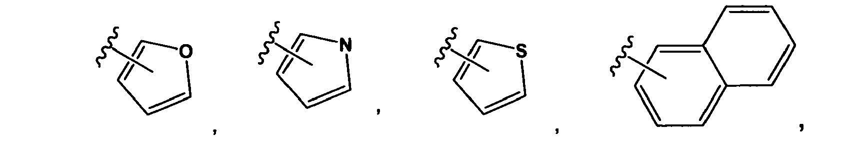 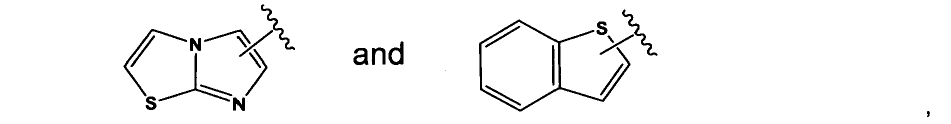 which is unsubstituted or optionally substituted in any suitable position with 1, 2, 3, 4 or 5 substituent(s) independently selected from the group consisting of oxo (=O), methyl, ethyl, n-propyl, isopropyl, n-butyl, tert-butyl, sec-butyl, phenyl, phenethyl, benzyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃, whereby the cyclic part(s) of said substituent(s) are unsubstituted or optionally substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂;
optionally in the form of one of its stereoisomers, preferably enantiomers or di-astereomers, its racemate or in the form of a mixture of at least two of its stereoisomers, enantiomers or diastereomers, in any mixture ratio, or a salt, a corresponding, physiologically acceptable salt, or a corresponding solvate.

4. Compounds according to one or more of claims 1 to 3,
wherein
R¹, R², R³ and R⁴, independent from one another, each represents H; F; Cl; Br; I; -NO₂; -SH; -OH; -CN; -OCF₃; -SCF₃; -NH₂; -O-CH₃; -O-CH₃-CH₃; -S-CH₃; -S-CH₂-CH₃; -NH-CH₃ or -N(CH₃)₂;
R⁵ and R⁶, independent from one another, each represents a hydrogen atom or a methyl, ethyl or n-propyl moiety, which is unsubstituted or optionally substituted with 1, 2, 3, 4 or 5 substituent(s) independently selected from the group consisting of F, Cl, Br, I, -OH and -SH;
or
R⁵ and R⁶ together with the bridging nitrogen atom form a heterocyclic ring system selected from the group consisting of 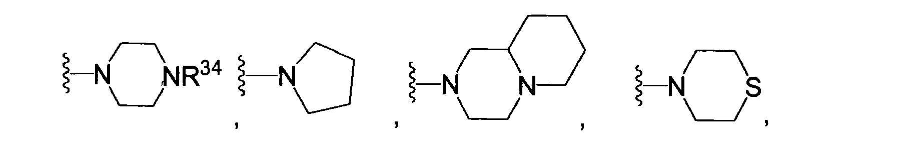 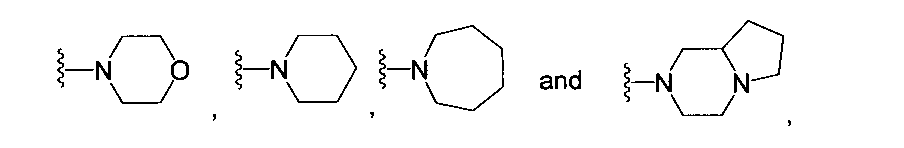 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, methyl, ethyl, phenyl, benzyl, phenethyl, -OCF₃, -SCF₃, -OH, -SH, -NH₂ and -O-CH₃;
R³⁴ represents a hydrogen atom; methyl; ethyl; n-propyl; isopropyl; phenyl; benzyl; phenethyl; -CF₃; -CHF₂; -CH₂F; -C(=O)-CH₃; -C(=O)-C₂H₅; -C(=O)-CH₂-CH₂-CH₃; -C(=O)-NH-CH₃ or a -C(=O)-N(CH₃)₂ moiety;
R⁷ represents a hydrogen atom; a methyl or an ethyl radical, which is unsubstituted or optionally substituted with 1, 2, 3, 4 or 5 substituent(s) independently selected from the group consisting of F, Cl, Br and I;
R⁸ represents a ring system selected from the group consisting of 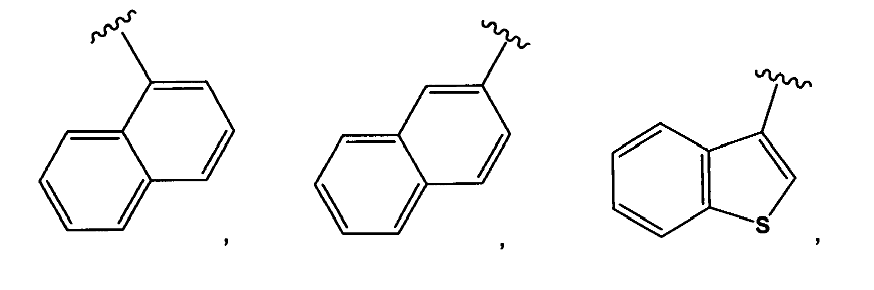 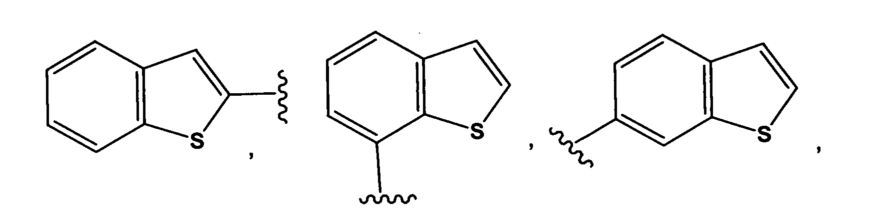 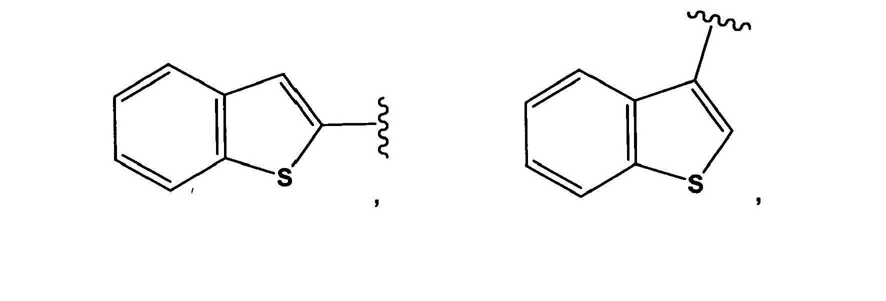 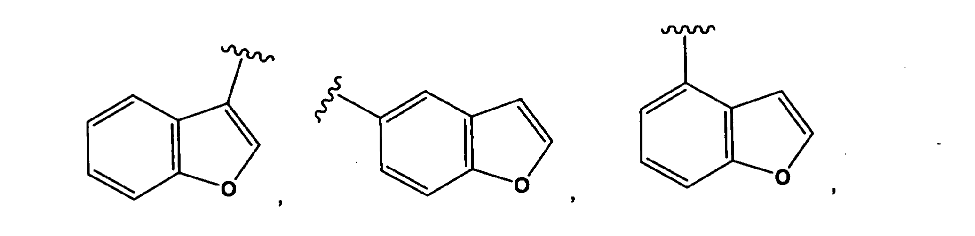 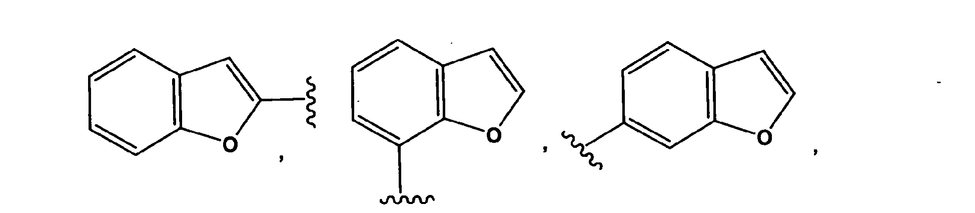 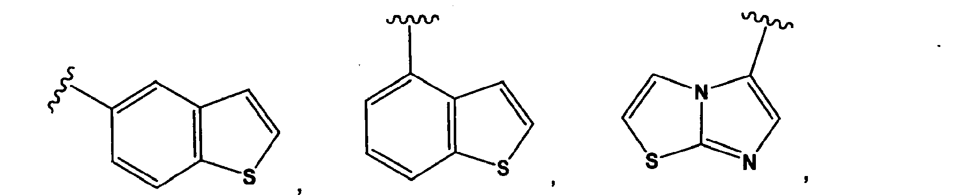 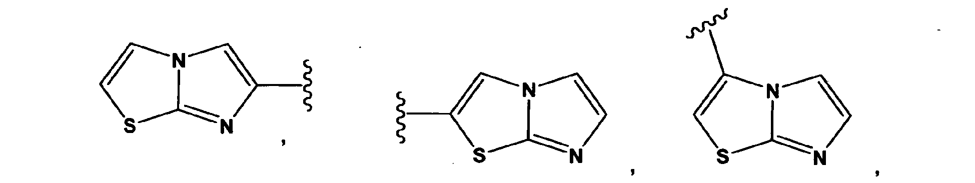 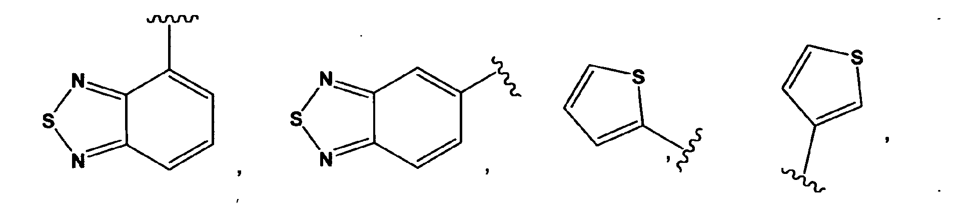 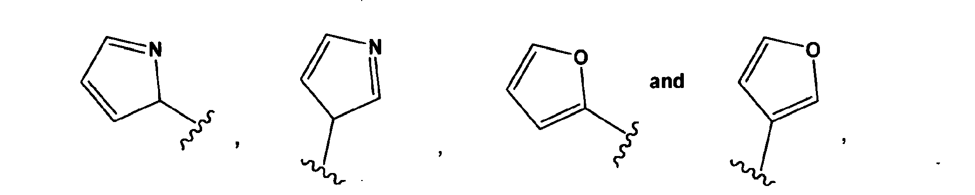 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, -methyl, -ethyl, -OCF₃, -SCF₃, -OH, -SH, -NH₂, -O-CH₃, -O-C₂H₅, -S-CH₃, -S-C₂H₅, -NH-CH₂, N(C₂H₅)₂, phenyl, phenethyl and benzyl, whereby the cyclic part(s) of said substituent(s) are unsubstituted or optionally substituted with 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

5. Compounds according to one or more of claims 1 to 4,
wherein
R¹, R², R³ and R⁴, independent from one another, each represents H; F; Cl; Br; I; - NO₂; -SH; -OH or -CN;
R⁵ and R⁶, independent from one another, each represents a hydrogen atom; a methyl, ethyl or n-propyl moiety which is unsubstituted or optionally substituted with 1, 2, 3, 4 or 5 substituent(s) independently selected from the group consisting of F, Cl, Br, I, -OH and -SH;
or
R⁵ and R⁶ together with the bridging nitrogen atom form a heterocyclic ring system selected from the group consisting of: 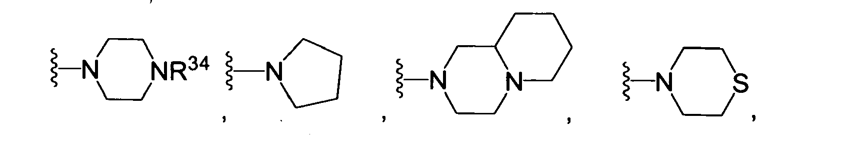 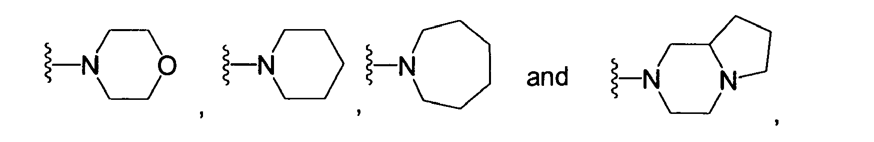 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, methyl, ethyl, phenyl, benzyl, phenethyl, -OCF₃, -SCF₃, -OH, -SH, -NH₂ and -O-CH₃,
R³⁴ represents a hydrogen atom, a methyl, ethyl or n-propyl radical;
R⁷ represents a hydrogen atom, a methyl or an ethyl radical, which is unsubstituted or optionally substituted with 1, 2 or 3 substituent(s) independently selected from the group consisting of F, Cl, Br and I;
R⁸ represents a ring system selected from the group consisting of: 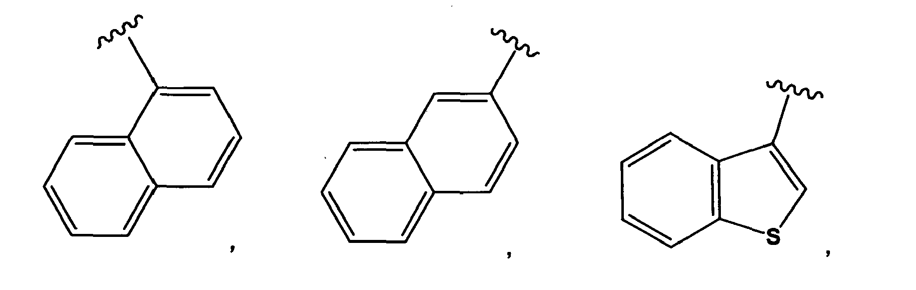 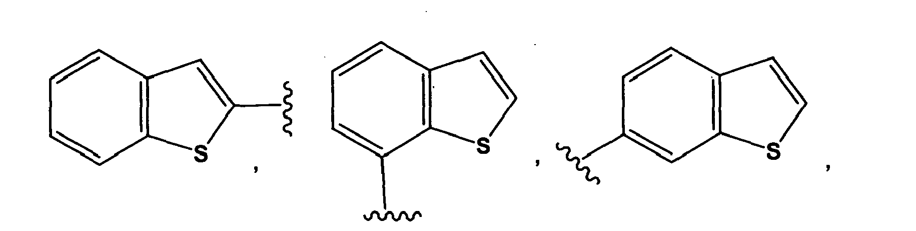 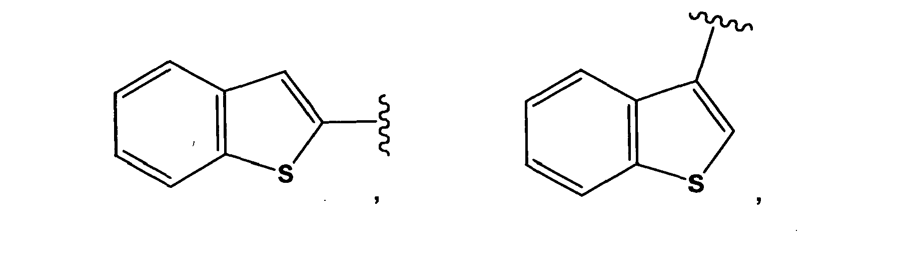 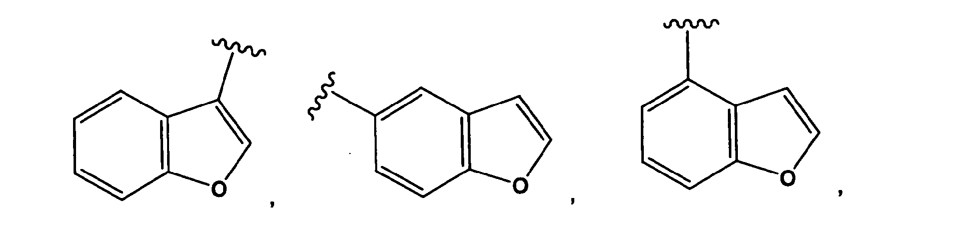 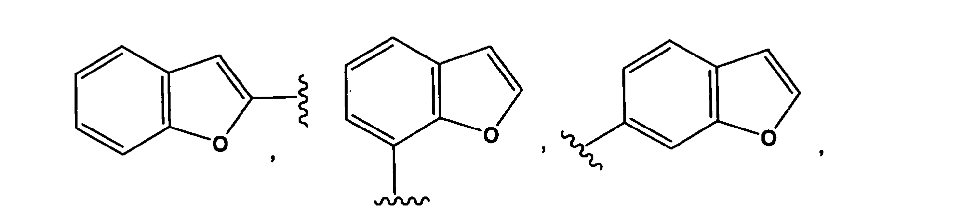 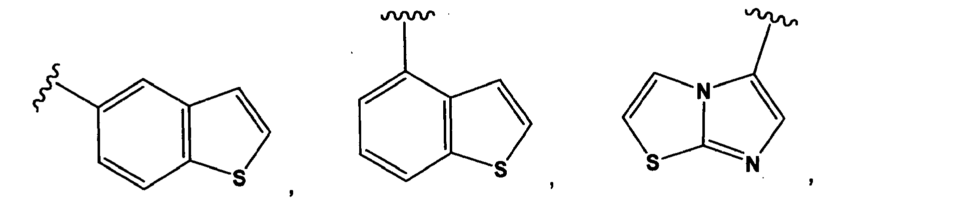 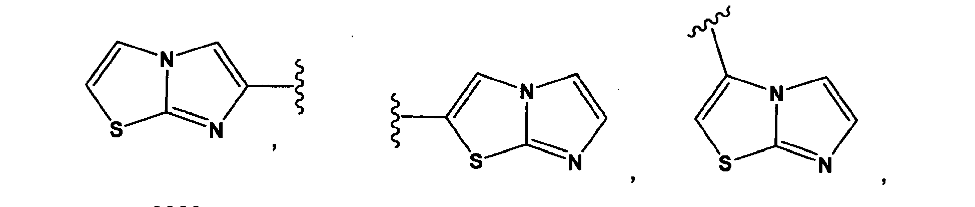 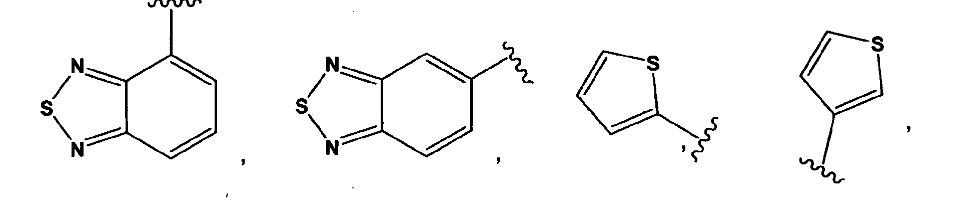 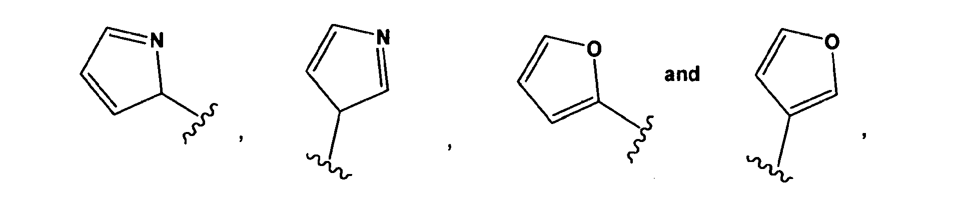 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, methyl, ethyl, phenyl, phenethyl, benzyl, -OCF₃, -SCF₃, -OH, -SH, -NH₂, -O-CH₃, -O-C₂H₅, -S-CH₃, -S-C₂H₅, -NH-CH₂ and -N(C₂H₅)₂;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

6. Compounds of general formula (Ia) according to any of claims 1 to 5, 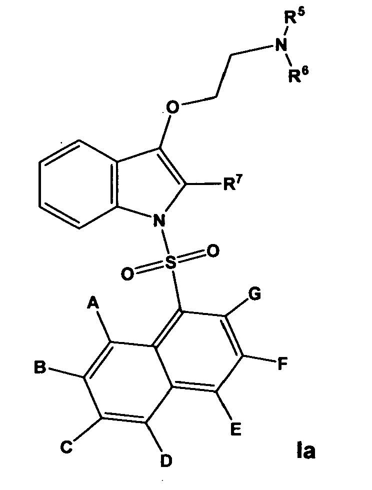 wherein
R⁵ and R⁶, independent from one another, each represents a hydrogen atom; a methyl, ethyl or n-propyl radical, which is unsubstituted or optionally substituted with 1, 2, 3, 4 or 5 substituent(s) independently selected from the group consisting of F, Cl, Br, I, -OH and -SH;
or
R⁵ and R⁶ together with the bridging nitrogen atom form a heterocyclic ring system selected from the group consisting of 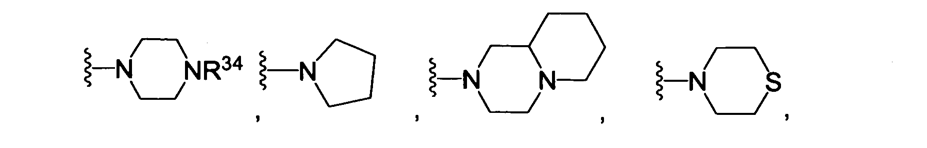 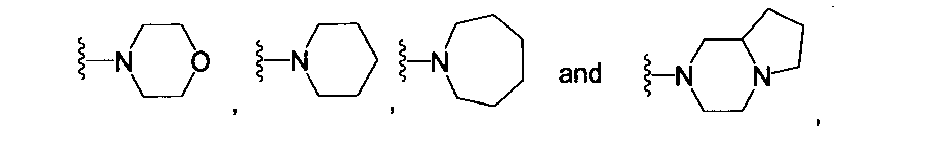 which is unsubstituted or optionally substituted in any suitable position with 1, 2, 3, 4 or 5 substituent(s) independently selected from the group consisting of F, Cl, Br, I, -CN, -CF₃, -CHF₂, -CH₂F, methyl, ethyl, phenyl, benzyl, phenethyl, -OCF₃, -SCF₃, -OH, -SH, -NH₂ and -O-CH₃,
R³⁴ represents a hydrogen atom, a methyl, ethyl or n-propyl radical;
R⁷ represents a hydrogen atom; a methyl or an ethyl radical, which is unsubstituted or optionally substituted with 1, 2, or 3 substituent(s) independently selected from the group consisting of F, Cl, Br and I;
A, B, C, D, E, F and G, independent from one another, each represents H; F; Cl; Br; I; -CN; -CF₃; -CHF₂; -CH₂F; methyl; ethyl; -OCF₃; -SCF₃; -OH; -SH; -NH₂; -O-CH₃; phenyl; benzyl or phenethyl;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

7. Compounds of general formula (Ib) according to any of claims 1 to 5, 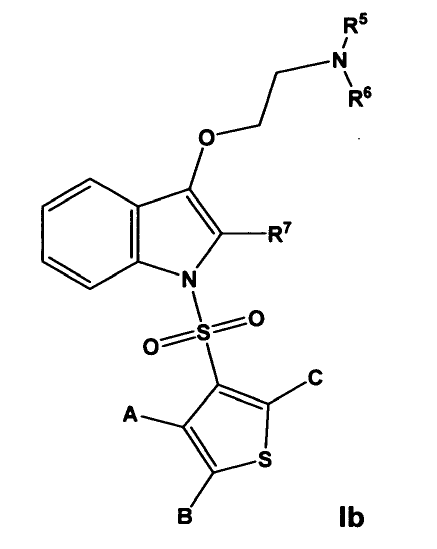 wherein
R⁵, R⁶, R⁷ and R³⁴ have the meaning according to claim 6;
A, B, and C have the meaning according to claim 6;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

8. Compounds of general formula (Ic) according to any of claims 1 to 5, 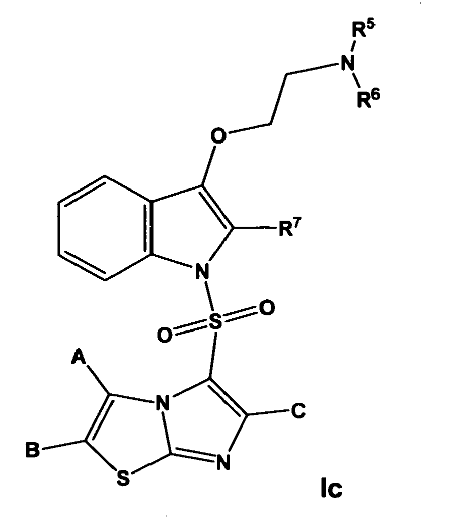 wherein
R⁵, R⁶, R⁷ and R³⁴ have the meaning according to claim 6;
A, B, and C have the meaning according to claim 6;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

9. Compounds of general formula (Id) according to any of claims 1 to 5, 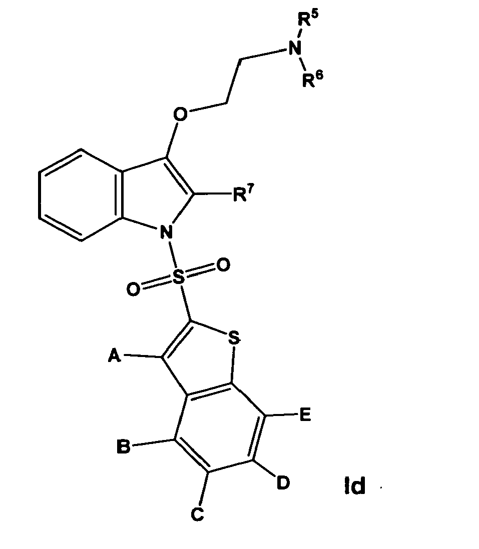 wherein
R⁵, R⁶, R⁷ and R³⁴ have the meaning according to claim 6;
A, B, C, D and E have the meaning according to claim 6;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

10. Compounds of general formula (Ie) according to any of claims 1 to 5, 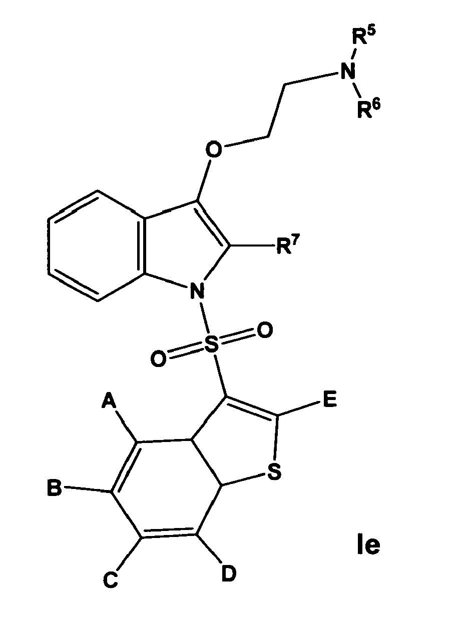 wherein
R⁵, R⁶, R⁷ and R³⁴ have the meaning according to claim 6;
A, B, C, D, E have the meaning according to claim 6;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

11. Compounds of general formula (If) according to any of claims 1 to 5, 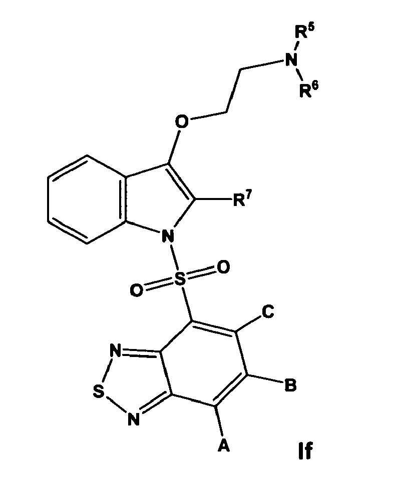 wherein
R⁵, R⁶, R⁷ and R³⁴ have the meaning according to claim 6;
A, B, and C have the meaning according to claim 6;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

12. Compounds of general formula (Ig) according to any of claims 1 to 5, 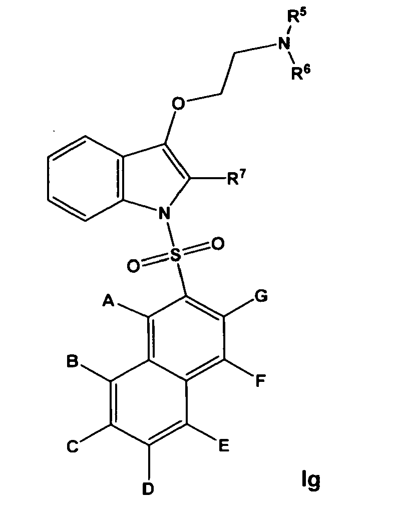 wherein
R⁵, R⁶, R⁷ and R³⁴ have the meaning according to claim 6;
A, B, C, D, E, F and G have the meaning according to claim 6;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

13. Compounds of general formula (Ih) according to any of claims 1 to 12, 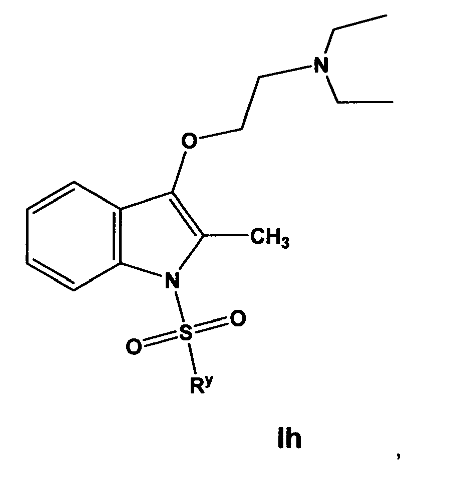 wherein
R^{y} represents a rings system selected from the group consisting of 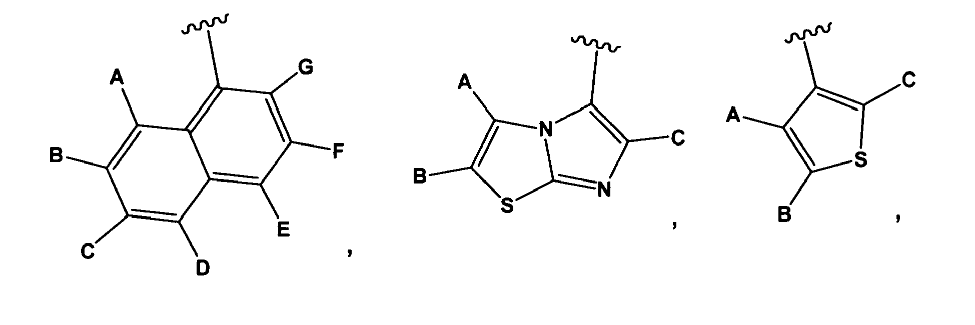 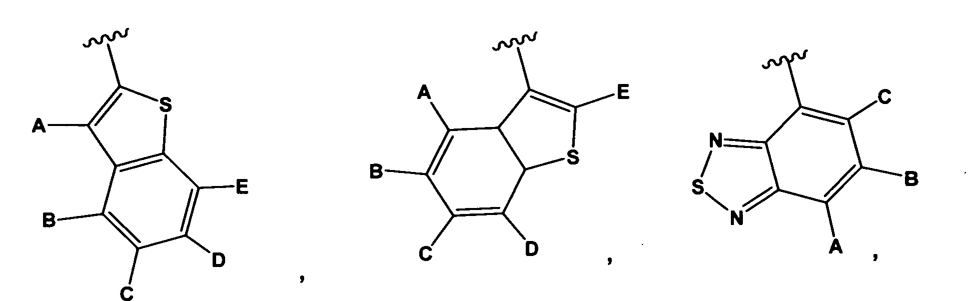 and 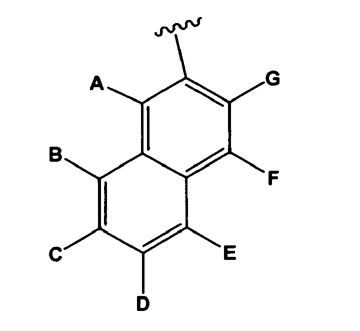
A, B, C, D, E, F and G, independent from one another, each represents H; F; Cl; Br; I; -CN; -CF₃; -CHF₂; -CH₂F; methyl; ethyl; -OCF₃; -SCF₃; -OH; -SH; -NH₂; -O-CH₃; phenyl; benzyl or phenethyl;
optionally in the form of one of its stereoisomers, preferably enantiomers or diastereomers, its racemate or in the form of a mixture of at least two of its stereoisomers, enantiomers or diastereomers, in any mixture ratio, or a salt, a corresponding, physiologically acceptable salt, or a corresponding solvate.

14. Compounds according to any of claims 1 to 13, selected from the group consisting of:
[1] N,N-diethyl-2-(2-methyl-1-(naphthalen-1-ylsulfonyl)-1H-indol-3-yloxy)ethanamine
[2] N,N-diethyl-2-(2-methyl-1-(naphthalen-2-ylsulfonyl)-1H-indol-3-yloxy)ethanamine
[3] 2-(1-(4-chlorothiophen-3-ylsulfonyl)-2-methyl-1H-indol-3-yloxy)-N,N-diethylethanamine
[4] 2-(1-(5-chloro-3-methylbenzo[b]thiophen-2-ylsulfonyl)-2-methyl-1H-indol-3-yloxy)-N,N-diethylethanamine
[5] 2-(1-(3a,7a-dihydrobenzo[b]thiophen-3-ylsulfonyl)-2-methyl-1H-indol-3-yloxy)-N, N-diethylethanamine
[6] 2-(1-(benzo[c][1,2,5]thiadiazol-4-ylsulfonyl)-2-methyl-1H-indol-3-yloxy)-N,N-diethylethanamine
[7] 2-(1-(6-chloroimidazo[2,1-b]thiazol-5-ylsulfonyl)-2-methyl-1H-indol-3-yloxy)-N,N-diethylethanamine,
and their corresponding salts and/or solvates.

15. A process for the preparation of a compound according to one or more of claims 1 to 14, **characterized in that** at least one compound of general formula (VII) 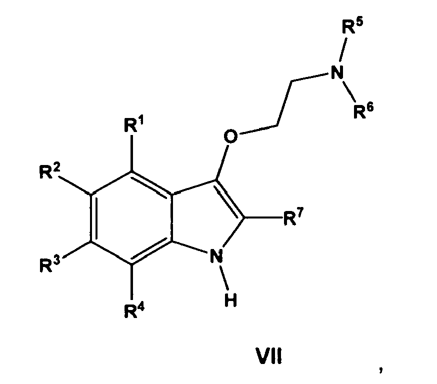 wherein R¹, R², R³, R⁴, R⁵, R⁶ and R⁷ have the meaning according to one or more of claims 1 to 14 is reacted with at least one compound of general formula (VIII),
**R⁸SO₂X** **VIII**,
wherein R⁸ has the respective meaning according to one or more of claims 1 to 14 and X represents a leaving group preferably selected from the group consisting of Cl and Br, optionally in the presence of at least one base, preferably in the presence of at least one base selected from the group consisting of alkali metal hydrides, more preferably in the presence of NaH, to yield at least one compound of general formula (I), 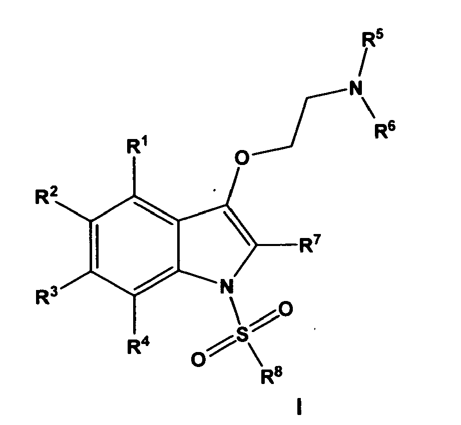 wherein R¹, R², R³, R⁴, R⁵, R⁶, R⁷ and R⁸ have the meaning according to one or more of claims 1 to 14.

16. A medicament comprising at least one substituted indole sulfonamide compound according to one or more of claims 1 to 14 and optionally at least one physiologically acceptable auxiliary agent.

17. A medicament according to claim 16 for the treatment and/or prophylaxis of diseases or disorders that are at least partially mediated via 5-HT₆ receptors.

18. A medicament according to claim 16 or 17 for the prophylaxis and/or treatment of food intake disorders, preferably for the regulation of appetite, for the maintenance, increase or reduction of body weight, for the prophylaxis and/or treatment of obesity, for the prophylaxis and/or treatment of bulimia, for the prophylaxis and/or treatment of anorexia, for the prophylaxis and/or treatment of cachexia, for the prophylaxis and/or treatment of type II diabetes (non-insulin dependent diabetes mellitus); for the prophylaxis and/or treatment of cognitive disorders, preferably memory disorders; or for improvement of cognition (for cognitive enhancement).

19. A medicament according to one or more of claims 16 or 17 for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia or for the prophylaxis and/or treatment hyperactivity disorder (ADHD, attention deficit, hyperactivity disorder).

20. Use of at least one substituted indole sulfonamide compound according to one or more of claims 1 to 14 for the manufacture of a medicament for 5-HT₆ receptor regulation.

21. Use of at least one substituted indole sulfonamide compound according to any one of claims 1 to 14 for the manufacture of a medicament for the prophylaxis and/or treatment of disorders that are at least partially mediated via 5-HT₆ receptors.

22. Use of at least one substituted indole sulfonamide compound according to any one of claims 1 to 14 for the manufacture of a medicament for the prophylaxis and/or treatment of food intake disorders; for the prophylaxis and/or treatment of cognitive disorders; or for improvement of cognition (for cognitive enhancement).

23. Use according to claim 22 for the regulation of appetite, for the reduction, increase or maintenance of body weight; for the prophylaxis and/or treatment of obesity, for the prophylaxis and/or treatment of bulimia, for the prophylaxis and/or treatment of anorexia; for the prophylaxis and/or treatment of cachexia; for the prophylaxis and/or treatment of type II diabetes; or for the prophylaxis and/or treatment of memory disorders.

24. Use of at least one substituted indole sulfonamide compound according to any one of claims 1 to 14 for the manufacture of a medicament for the prophylaxis and/or treatment of gastrointestinal disorders, preferably irritable colon syndrome; for the prophylaxis and/or treatment of disorders of the central nervous system; for the prophylaxis and/or treatment of anxiety; for the prophylaxis and/or treatment panic attacks; for the prophylaxis and/or treatment of depression; for the prophylaxis and/or treatment of bipolar disorders; for the prophylaxis and/or treatment of senile dementia; for the prophylaxis and/or treatment of psychosis; for the prophylaxis and/or treatment neurodegenerative disorders; preferably selected from the group consisting of Morbus Alzheimer, Morbus Parkinson, Morbus Huntington and Multiple Sclerosis; for the prophylaxis and/or treatment of schizophrenia; or for the prophylaxis and/or treatment hyperactivity disorder (ADHD, attention deficit, hyperactivity disorder).
